(19) Europäisches Patentamt
European Patent Office
Office européen des brevets

(11) **EP 2 888 363 B1**

(12) **EUROPEAN PATENT SPECIFICATION**

(45) Date of publication and mention
of the grant of the patent:
**06.06.2018   Bulletin 2018/23**

(51) Int Cl.:
***C12N 15/10*** *(2006.01)*          ***C12Q 1/68*** *(2018.01)*

(21) Application number: **13756018.1**

(86) International application number:
**PCT/EP2013/067351**

(22) Date of filing: **21.08.2013**

(87) International publication number:
**WO 2014/029791 (27.02.2014 Gazette 2014/09)**

(54) **METHOD FOR ISOLATING NUCLEIC ACIDS FROM A FORMALDEHYDE RELEASER STABILIZED SAMPLE**

VERFAHREN ZUR ISOLIERUNG VON NUKLEINSÄUREN AUS EINER STABILISIERTEN PROBE MIT FORMALDEHYDFREIGABE

PROCÉDÉ POUR ISOLER DES ACIDES NUCLÉIQUES À PARTIR D'UN ÉCHANTILLON STABILISÉ LIBÉRANT DU FORMALDÉHYDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.08.2012   EP 12181137**

(43) Date of publication of application:
**01.07.2015   Bulletin 2015/27**

(73) Proprietor: **Qiagen GmbH**
**40724 Hilden (DE)**

(72) Inventors:
• **GÜNTHER, Kalle**
  **40724 Hilden (DE)**
• **WYRICH, Ralf**
  **40724 Hilden (DE)**
• **OELMÜLLER, Uwe**
  **40724 Hilden (DE)**

(74) Representative: **Roth, Carla et al**
**König-Szynka-Tilmann-von Renesse**
**Patentanwälte Partnerschaft mbB**
**Mönchenwerther Straße 11**
**40545 Düsseldorf (DE)**

(56) References cited:
EP-A1- 2 395 082          WO-A1-2011/057184
US-A- 5 459 073           US-A- 5 460 797
US-A1- 2005 059 054       US-B2- 6 617 170
US-B2- 7 270 953

• **KALLE GÜNTHER ET AL: "Implementation of a proficiency testing for the assessment of the preanalytical phase of blood samples used for RNA based analysis", CLINICA CHIMICA ACTA, ELSEVIER BV, AMSTERDAM, NL, vol. 413, no. 7, 15 January 2012 (2012-01-15), pages 779-786, XP028463581, ISSN: 0009-8981, DOI: 10.1016/J.CCA.2012.01.015 [retrieved on 2012-01-21]**

Remarks:
The file contains technical information submitted after the application was filed and not included in this specification

## Description

The work leading to this invention has received funding from the European Community's Seventh Framework Programme (*FP7/2007-2013)* under *grant agreement* n° 222916.

## FIELD OF THE INVENTION

This invention pertains to the isolation of nucleic acids from samples that were stabilised using a formaldehyde releaser.

## BACKGROUND OF THE INVENTION

Nucleic acids are important biomarkers in the diagnostic field. Profiles of transcripts of the genome (in particular mRNA and miRNA) are widely used as biomarkers in molecular *in vitro* diagnostics and promised to provide inside into normal biological and pathological processes with the hope of predicting disease outcome and indicating individualised courses of therapy. Therefore, profiling of nucleic acids, in particular RNA, is becoming important in disease diagnosis, prognosis and in clinical trials for biomarker discovery. The ability to obtain quantitative information from the transcriptional profile would thus be a powerful tool to explore basic biology, diagnose disease, facilitate drug development, tailor therapeutics to specific pathologies and genetic profiles and also to generate databases relevant to biological or therapeutic processes and pathways. Significant improvements of downstream assays and data analyses (analytical process) have been made during the last years. However, it was found that the preanalytical steps, such as sample stabilisation, in particular for new biomolecular targets, have a severe impact on the expression profile and may compromise the subsequent analysis (see for example Härtel et al, 2001, Pahl und Brune, 2002). Without precaution in the stabilisation of the sample to be analysed, the sample will undergo changes during transport and storage that may alter the expression profile of the targeted molecules (see for example Rainen et al, 2002; Baechler et al, 2004). If the expression profile is altered due to the handling of the sample, the subsequent analysis does not reflect the original situation of the sample and hence of the patient but rather measure an artificial profile generated during sample handling, transport and storage. Therefore, optimized stabilisation processes are needed which stabilise the expression profile.

Stabilisation of samples such as in particular blood samples of a longer period was formally performed with the addition of organic solvents such as phenol and/or chloroform or by direct freezing in liquid nitrogen or using dry ice. These methods are not at all practicable techniques for hospitals, doctor surgeries or diagnostic routine laboratories. To overcome these problems, PreAnalytiX developed the first research product for the collection of human blood with an evacuated blood collection tube that contains reagents for an immediate stabilisation of the RNA gene expression profile at the point of sample collection. The respective stabilisation composition allows the transport and storage at room temperature without the risk of changes in the RNA profile by gene induction and transcript degradation (see for example US 6,617,170, US 7,270,953, Kruhoffer et al, 2007). The respective compositions are sold under the name of PAXgene Blood RNA Tubes.

Similar stabilisation agents that achieve an immediate lysis of the sample are sold by ABI/Life Technologies with the Tempus Blood RNA tube product. The tubes also comprise a stabilisation reagent and the sample, here blood, that is drawn into the tube and mixed with this stabilization reagent is immediately lysed and cellular RNases are inactivated and nucleic acids are precipitated. The vast majority of proteins remains in solution.

The disadvantage of the respective methods is that the stabilisation results in the complete lysis of the cells, thereby destroying the cells morphology. However, not only the quality and quantity of the isolated nucleic acids respectively their expression profile is of analytical interest, but also the presence, absence or number of specific cells contained in the sample such as for example a blood sample. The destruction of the cells is a great disadvantage because any cell sorting or cell enrichment respectively cell analysis becomes impossible. This is a disadvantage with respect to the sensitivity of the detection of, for example, circulating tumor cells in the given background of normal white blood cells.

Therefore, very often specific stabilisation reagents, respectively blood collection tubes are provided that are specifically intended for the stabilisation of cells. The respective products allow to investigate the cellular content of the sample after storage, for example to detect the presence of tumor cells for example by fluorescence activated cell sorting (FACS) analysis or changes of the ratio of different white blood cells to each other by flow cytometry (FC) or FACS analysis. E.g. many workflows use standard EDTA blood collection tubes for flow cytometry or FACS analysis, although blood cells show minor lysis over time of storage. A further product from Streck Inc. is a direct-draw vacuum blood collection tube for the preservation of whole blood samples for immunophenotyping by flow cytometry. It preserves white blood cell antigens allowing subsets of leucocytes to be distinguished by flow cytometry analysis. The technology to maintain the integrity of the white blood cell cluster of differentiation (CD) markers is e.g. covered by US 5,460,797 and US 5,459,073.

However, using different stabilisation reagents and accordingly stabilisation tubes for collecting the sample for nucleic acid analysis and cell analysis is tedious. There is a need to reduce the number of different sample collection tubes, for example blood collection tubes, per draw at the patients' site that are dedicated to different downstream assays (e.g. detection of cells and analysis of RNA).

Therefore, sample collection and stabilisation systems are needed, which keep the cell's morphology intact while at the same time stabilising the nucleic acids. Respective stabilisation systems are advantageous for example in the molecular diagnostic of cancer, because they would make an enrichment of cells prior to the extraction of the nucleic acids from the cells possible and would thereby increase the chance to detect rare events of circulating tumor cells in the samples, for example a blood sample. This would increase the chance that a specific biomarker is identified in the sample.

To address the need of simultaneous cell stabilisation and nucleic acid stabilisation, stabilisation systems were developed that are based on the use of formaldehyde releasers. Respective stabilisation agents are commercially available from Streck Inc. under the name of cell-free RNA BCT (blood collection tube). The 10 ml blood collection tube is intended for the preservation and stabilisation of cell-free RNA in plasma for up to 3 days at room temperature. The preservative stabilizes cell-free RNA in plasma and prevents the release of non-target background RNA from blood cells during sample processing and storage.

Furthermore, a patent application was published that describes the use of formaldehyde releasing components to achieve cell and RNA stabilisation in the same blood sample (US 2011/0111410). Therefore, this document describes a technique wherein the stabilisation agent stabilises the blood cells in the drawn blood thereby preventing contamination of cellular RNA with cell-free RNA or globin RNA, inhibits the RNA synthesis for at least 2 hours and cellular RNA that is within the blood cells is preserved to keep the protein expression pattern of the blood cells substantially unchanged to the time of the blood draw. The white blood cells can be isolated from the respectively stabilised sample and cellular RNA is than extracted from the white blood cells. With respect to the nucleic acids isolation protocol that can be used to isolate nucleic acids from respectively stabilised samples the application refers to commercial nucleic acid isolation products such as the AllPrep DNA/RNA mini Kit (QIAGEN).

However, nucleic acid isolation from respectively stabilised samples is very difficult, because the used formaldehyde releaser interferes with the subsequent nucleic acid isolation process. Therefore, the nucleic acid yield and/or purity is severely reduced compared to the isolation of nucleic acids that were stabilised using stabilization methods that specifically aim at the stabilization and isolation of nucleic acids such as RNA (for example the PAXgene Blood RNA Tubes).

The object of the present invention is to improve the isolation of nucleic acids from stabilised samples and in particular from samples that were stabilised using a formaldehyde releaser.

## SUMMARY OF THE INVENTION

The present invention is based on the finding that the isolation of nucleic acids from a sample that has been stabilized by the use of a formaldehyde releaser can be greatly improved if a cationic detergent is used during lysis of the stabilized sample. Thereby, the yield and purity of the isolated nucleic acids, in particular RNA, can be greatly improved.

Thus, according to a first aspect, the present disclosure pertains to a method for isolating nucleic acids from a stabilized sample or portion or fraction thereof, wherein the sample stabilization involved the use of at least one formaldehyde releaser and wherein the isolation of the nucleic acids from the stabilized sample or portion or fraction thereof involves the use of at least one cationic detergent during lysis.

According to a second aspect, the present disclosure pertains to the use of a cationic detergent during lysis of a stabilized sample or portion or fraction thereof in preparation for nucleic acid isolation, wherein the sample stabilization involved the use of at least one formaldehyde releaser.

Other objects, features, advantages and aspects of the present application will become apparent to those skilled in the art from the following description and appended claims. It should be understood, however, that the following description, appended claims, and specific examples, while indicating preferred embodiments of the application, are given by way of illustration only. Various changes and modifications within the spirit and scope of the disclosed invention will become readily apparent to those skilled in the art from reading the following.

## DETAILED DESCRIPTION OF THIS INVENTION

The present disclosure pertains to the use of at least one cationic detergent during lysis of a stabilized sample or portion or fraction thereof in preparation for nucleic acid isolation, wherein the sample stabilization involved the use of at least one formaldehyde releaser.

The present invention is defined in the claims.

The method according to the present invention is for the isolation of nucleic acids from specifically stabilized samples, namely samples that were stabilized by using one or more formaldehyde releasers. Isolating nucleic acids from respectively stabilized samples is particularly challenging, because the stabilization severely reduces the amount of nucleic acids that can be isolated from the stabilized sample. As is shown by the examples, the nucleic acid isolation from such samples can be considerably improved with respect to yield and purity, if a cationic detergent is used for lysis. Subsequently, we will explain the individual steps and preferred embodiments of the present invention:

First, a stabilized sample is obtained, wherein the sample stabilization involved the use of at least one formaldehyde releaser. Respectively stabilized samples may be obtained, e.g. received from a facility wherein the sample was collected, such as e.g. a hospital. However, the stabilization and nucleic acid isolation may also be performed at the same facility. In this case, the sample is stabilized upon collection by contacting the sample with a stabilization composition comprising a formaldehyde releaser. Respective stabilization methods that are based on the use of a formaldehyde releaser are known in the prior art and are e.g. disclosed in US 2011/0111410. A stabilisation method using a formaldehyde releaser has several advantages. It efficiently preserves contained nucleic acids such as RNA and DNA but also cells if comprised. The stabilization which is based on the use of at least one formaldehyde releaser results in short term inhibition of the cell metabolism (in partiuclar RNA synthesis) and results in a stabilization of cellular RNA within the cells. Thereby, the mRNA expression pattern, also referred to as transcriptom, is immediately stabilized at the time stabilization occurs which usually occurs upon collection of the samples. Furthermore, the RNA is protected from degrading enzyme such as nucleases. Additionally, the cells are preserved in the sample and cell lysis is inhibited due to said stabilization technology that involves a formaldehyde releaser. This prevents that nucleic acids located outside the cells, also referred to extracellular nucleic acids, become contaminated with intracellular nucleic acids that otherwise would be released from lysed cells. Furthermore, proteins are also stabilized. It was shown by various tests that the stabilization involving the use of formaldehyde releaser preserves the surface structure and also the surface proteins of cells contained in the sample. This is a further advantage. Therefore, a stabilization method which is based on the use of a formaldehyde releaser results in an efficient preservation of the sample and therefore reflects the status of the sample at the time of collection, respectively stabilization.

Thus, the stabilisation method that is used in the method according to the present invention which does not rely on the use of stabilization agents that destroy the comprised cells also allows to sort or capture cells from the sample to enrich specific cell types, for example tumor cells, or cells contained in the sample such as white blood cells, that further on can be used to isolate the nucleic acids in particular the RNA from these specific cells. As the nucleic acids would then be derived not from all but only from specific cell types of interest contained in the sample, the target nucleic acids such as RNA molecules, would be enriched. Furthermore, cell profile specific analysis such as reverse transcription amplification based analysis become possible. This is a great advantage when for example intending to analyse tumor cell specific transcripts. This increases the usability, sensitivity and accuracy of the downstream assays.

The sample stabilization involves the use of a stabilizing composition which comprises one or more formaldehyde releasers. A formaldehyde releaser as used herein is a compound which over time releases formaldehyde and/or paraformaldehyde. Thus, formaldehyde and/or paraformaldehyde is slowly released. Respective stabilization methods are known in the prior art. Exemplary and preferred embodiments of a respective stabilization which can be used in conjunction with the present invention are described in the following. The formaldehyde releaser acts as preservative agent. The formaldehyde releaser may be a chemical fixative. Suitable formaldehyde releasers include but are not limited to, diazolidinyl urea, imidazolidinyl urea, dimethoylol-5,5dimethylhydantoin, dimethylol urea, 2-bromo-2.- nitropropane-1,3-diol, oxazolidines, sodium hydroxymethyl glycinate, 5- hydroxymethoxymethyl-1 1 -1 aza-3,7-dioxabicyclo[3.3.0]octane, 5-hydroxymethyl-1 - 1 aza-3,7dioxabicyclo[3.3.0]octane, 5-hydroxypoly[methyleneoxy]methyl-1 1-1 aza-3, 7dioxabicyclo[3.3.0]octane, quaternary adamantine or any combinations of the foregoing. The formaldehyde releaser preferably is a heterocyclic urea and may be selected from the group consisting of diazolidinyl urea (DU), imidazolidinyl urea (IDU), and any combination thereof. In advantageous embodiments, the stabilization involved the use of diazolidinyl urea (DU) and/or imidazolidinyl urea, preferably diazolidinyl urea.

The use of a formaldehyde releaser to stabilize a sample has compared to typical histological fixing agents such as e.g. formaldehyde the advantage that the toxicity is very low. Furthermore, it is assumed that formaldehyde releasers stabilize the sample differently than other fixatives such as formaldehyde. This can be seen in the characteristics of the stabilized sample. In particular, the formaldehyde releaser is more efficient in stabilizing RNA and additionally, the isolation of cells using standard methods such as flow cytometry, in particular FACS, is possible from samples that were stabilized with a formaldehyde releaser. This is advantageous when intending to isolate cells from the stabilized sample which is often desirous for analytical and in particular diagnostic purposes. It is thus preferred that the stabilizing composition is non-toxic and preferably free of separately added formaldehyde and/or separately added paraformaldehyde. According to one embodiment, the whole stabilization of the sample did not involve the addition of formaldehyde or paraformaldehyde as chemical compounds. According to one embodiment, the stabilization of the sample did not involve the use of an additional fixative which cross-links proteins.

As is shown by the examples, already the formaldehyde releaser alone is highly effective in stabilizing biological samples. Therefore, the formaldehyde releaser can be used alone for stabilization purposes. However, depending on the type of sample and also the intended storage time, further additives may be contained in the stabilization composition or may be added separately to the sample to be stabilized in order to improve the stabilization effect. For example, when stabilizing blood or a sample derived from blood, the stabilization will involve the addition of an anticoagulant. Examples of anticoagulants that can be comprised in the stabilization composition or can be added separately to the sample include but are not limited to heparin, metal ion chelating agents, in particular citrate, oxalate, EDTA and combinations thereof. These and other anticoagulants are also well-known in the prior art. However, in order to achieve an immediate stabilization and to directly prevent the lysis of the contained cells it is preferred that the anticoagulant and the stabilization composition are either given at or approximately at the same time or, preferably, to include the anticoagulant in the stabilization composition which comprises the formaldehyde releaser.

Further suitable additives that may be added either separately to the sample or may form part of the stabilization composition are described in the following. These are of course non-limiting and accordingly, other additives may be included respectively added as well.

According to one embodiment, the stabilization composition that was used for stabilizing the sample or the stabilization process as such may have further comprised one or more additional additives. Said one or more additives may be selected from the group consisting of enzyme inhibitors, preferably nuclease inhibitors, more preferably RNase inhibitors, metabolic inhibitors, preferably glyceraldehyde, cell membrane permeabilisers, amino acids, protease inhibitors, phosphatase inhibitors, oxidative stress neutralizers such as e.g. antioxidants or reactive oxygen species and metal ion chelators. Preferably, two or more of the respective additives are comprised in the stabilization composition or are added separately during the stabilization process. Suitable examples are described below. Therein, we specifically refer to the embodiment, wherein the one or more optional additives are provided as constituent of the stabilization composition. However, as discussed above, these one or more optional additives may also be added separately. Thus, the subsequent disclosure equally applies to embodiments, wherein said optional additives are added separately from the formaldehyde releaser.

In advantageous embodiments, the stabilization composition that was used to provide the stabilized sample comprised one or more enzyme inhibitors like nuclease inhibitors in a suitable amount to prevent DNase and/or RNase activity from decreasing the quality and amount of nucleic acids recoverable from the sample. Nuclease inhibitors that may be used for stabilization include, but are not limited to diethyl pyrocarbonate, ethanol, aurintricarboxylic acid (ATA), formamide, vanadyl- ribonucleoside complexes, macaloid, ethylenediamine tetraacetic acid (EDTA), proteinase K, heparin, hydroxylamine-oxygen-cupric ion, bentonite, ammonium sulfate, dithiothreitol (DTT)1 beta-mercaptoethanol, cysteine, dithioerythritol, tris (2-carboxyethyl) phosphene hydrochloride, or a divalent cation such as $Mg^{2+}$, $Mn^{+2}$, $Zn^{+2}$, $Fe^{+2}$, $Ca^{2+}$, $Cu^{+2}$ and any combination thereof.

Preferred nuclease inhibitors inactivate nucleases (e.g. RNases) by binding to them so that the nucleases are no longer capable of contacting the nucleic acids comprised in the sample thereby reducing the adverse effects of nucleases on the quantity and quality of the nucleic acids. In an advantageous embodiment, the nuclease inhibitors used for stabilization include aurintricarboxylic acid (ATA) and/or metal ion complexing agents such as ethylenediamine tetraacetic acid (EDTA).

The stabilization composition used for stabilizing the sample may have also included one or more metabolic inhibitors in a suitable amount to reduce cell metabolism within a body fluid sample. Metabolic inhibitors that may be used include, but are not limited to glyceraldehyde, dihydroxyacetone phosphate, glyceraldehyde 3-phosphate, 1,3-bisphosphoglycerate, 3-phosphoglycerate, 2- phosphoglycerate, phosphoenolpyruvate, pyruvate and glycerate dihydroxyacetate, sodium fluoride, $K_2C_2O_4$ and any combination thereof. In advantageous embodiments, the metabolic inhibitor is sodium fluoride and/or glyceraldehyde.

The stabilization composition may have also included one or more metal ion chelators in a suitable amount, wherein the metal ion chelator is selected from the group consisting of ethylene glycol tetraacetic acid (EGTA), 1 ,2-bis-(o-Aminophenoxy)-ethane-N',N',- N',N'-tetraacetic acid tetraacetoxy-Methyl ester (BAPTA-AM), diethyldithiocarbamate (DEDTC), ethylenediaminetetraacetic acid (EDTA), dicarboxymethyl- glutamic acid, nitrilotriacetic acid (NTA), ethylenediaminedisuccinic acid (EDDS), polycarboxylic acids, citrate and any combination thereof. In advantageous embodiments, the metal ion chelator is EDTA. As described above, metal ion chelators may function as nuclease inhibitors. Furthermore, and this is particularly advantageous when stabilizing a blood sample or a sample derived from blood, metal ion chelators can function as anticoagulant. The metal ion chelator is preferably comprised in a concentration so that it acts as anticoagulant, in particular if the sample is a blood sample.

Preferably, the stabilization composition is an aqueous composition, preferably an aqueous solution. The stabilization composition that was used for providing the stabilized sample may have comprised suitable solvents such as water, buffers (e.g. MOPS, TRIS, PBS and the like), saline, dimethylsulfoxide, alcohol and any mixture thereof. According to one embodiment, the stabilization composition which preferably is a solution comprises/has comprised diazolidinyl urea and/or imidazolidinyl urea in a buffered salt solution. The stabilization composition which preferably is a solution may have further comprised an anticoagulant, preferably EDTA. The stabilization composition solution may also comprise/have comprised one or more metabolic inhibitors, preferably sodium fluoride and/or glyceraldehyde and/or a nuclease inhibitor.

It is within the scope of the present invention that the stabilization composition is not in a liquid but in a solid form. For example, all or selected components of the stabilization composition may undergo a lyophilisation process so that each component is added either prior to or post collection in a substantially solid form. This may help e.g. to prevent unwanted reactions between one or more components of the stabilization composition. Liquid removal techniques can be performed on the stabilization composition in order to obtain a substantially solid state stabilization composition. Liquid removal conditions may preferably be such that they result in removal of at least about 50 percent by weight, more preferable about 75 percent by weight, and still more preferably at least about 85 percent by weight of the original amount of the dispensed liquid stabilization composition. Liquid removal conditions may preferably be such that they result in removing of sufficient liquid so that the resulting composition is in the form of a film, gel or other substantial solid or highly viscous layer, for example it may result in a substantially immobile coating (preferably a coating that can be re-dissolved or otherwise dispersed upon contact with the sample to be stabilized). Thus, liquid removal conditions may preferably be such that they result in a material that upon contact with the sample under consideration the stabilization composition in its constituents will disperse in the sample, and substantially preserve the components in the sample, such as in particular the cells and contained nucleic acids. Liquid removal conditions may preferably be such that they result in a remaining composition that is substantially free of crystallinity, has a viscosity that is sufficiently high and that the remaining composition is substantially immobile at ambient temperature. Respective liquid removal techniques are well known to the skilled person and thus, do not need a detailed description here.

The amount of any active ingredient within the stabilization composition and in particular the amount of formaldehyde releaser necessary to achieve a sufficient stabilization of a specific sample may vary from sample to sample and in particular depends on the characteristics of the sample, in particular the amount of the comprised cells. Suitable concentrations can be determined by skilled person and only require routine experiments. Suitable concentration ranges for liquid stabilization compositions are given in the following. These compositions are particularly suitable for stabilising body fluids such as blood. Any percentages given by weight refer in case of a liquid composition to weight per volume.

The amount of any active ingredient within the stabilization composition may generally be at least about 0.01 percent by weight. The amount of any active ingredient within the stabilization composition may generally be 90 percent by weight or less, 85 percent by weight or less, 80 percent by weight or less, 75 percent by weight or less, 70 percent by weight or less, 60 percent by weight or less, 50 percent by weight or less or 40 percent by weight or less. The stabilization composition may comprise at least about 10 percent by weight, preferably at least about 15 percent by weight, more preferred at least 20 percent by weight formaldehyde releaser, which preferably is diazolidinyl urea. The stabilization composition may comprise 90 percent by weight or less, 85 percent by weight or less, 80 percent by weight or less, 75 percent by weight or less, 70 percent by weight or less, 65 percent by weight or less, 60 percent by weight or less, 55 percent by weight or less, 50 percent by weight or less, 45 percent by weight or less or 40 percent by weight or less formaldehyde releaser which preferably is diazolidinyl urea. The stabilization composition may further contain at least about 0.1 percent by weight, at least 0.5 percent by weight or at least 1 percent by weight of one or more enzyme inhibitors (e.g., nuclease inhibitors) such as EDTA and ATA. The stabilization composition may contain less than about 40 percent by weight or less than about 30 percent by weight of one or more enzyme inhibitors, such as a nuclease inhibitor. The stabilization composition may also contain at least about 1 percent by weight, preferably at least about 2 percent by weight of one or more metabolic inhibitors. The stabilization composition may contain 40 percent by weight or less, 30 percent by weight or less, 20 percent by weight or less, 15 percent by weight or less, or 10 percent by weight or less of one or more metabolic inhibitors. Preferably, glyceraldehyde is used as metabolic inhibitor.

In advantageous embodiments, the stabilization composition that was used for providing the stabilized sample comprises a formaldehyde releaser in a concentration selected from 10 to 70 percent by weight, 10 to 60 percent by weight or 10- 50 percent by weight, preferably 15 to 40 percent by weight, more preferred 20- 30 percent by weight. Preferably, diazolidinyl urea and/or imidazolidinyl urea is used as formaldehyde releaser. In another advantageous embodiment the stabilization composition comprises in addition to the formaldehyde releaser an enzyme inhibitor in a concentration selected from 0.05 to 40 percent by weight, 0.1 to 30 percent by weight, 0.1 to 20 percent by weight, 0.1 to 10 percent by weight or 0.1 to 5 percent by weight, preferably 0.5 to 3 percent by weight, more preferred 0.5- 2.5 percent by weight. In one embodiment ATA is used as enzyme inhibitor. In a further advantageous embodiment the stabilization composition comprises in addition to the formaldehyde releaser and optionally in addition to the enzyme inhibitor at least one metabolic inhibitor in a concentration selected from 0.05 to 40 percent by weight, 0.05 to 30 percent by weight, 0.05 to 20 percent by weight, 0.05 to 15 percent by weight, 0.05 to 12.5 percent by weight or 0.05 and 8 percent by weight, preferably glyceraldehyde in a concentration selected from 1 - 8 percent by weight and/or sodium fluoride in a concentration selected from 0.05- 1.5 percent by weight. In other advantageous embodiments the stabilization composition comprises in addition to the formaldehyde releaser and optionally in addition to the enzyme inhibitor and/or the metabolic inhibitor a metal ion chelator in a concentration selected from 0.1 to 40 percent by weight, 1 to 30 percent by weight, 2.5 to 25 percent by weight, 5 percent to 20 percent by weight, preferably 5-15 percent by weight.

In embodiments according to the present disclosure, the stabilization composition that was used for providing the stabilized sample comprises

a) a formaldehyde releaser selected from a chemical fixative that contains urea, preferably diazolidinyl urea and/or imidazolidinyl urea,

and in addition thereto, at least one, preferably at least two, preferably all of the following components:

b) an enzyme inhibitor, preferably aurintricarboxylic acid,

c) a metabolic inhibitor, preferably glyceraldehyde and/or sodium fluoride, and/or

d) a metal ion chelator, preferably EDTA.

Suitable and preferred concentration ranges for the respective components were described above. Said stabilization composition is when comprising a metal ion chelator particularly suitable for stabilizing body fluids, in particular whole blood samples or samples derived from blood.

The stabilization composition is contacted with the sample for stabilization. The amount of stabilization composition to be added to the sample to achieve stabilization depends on the type of sample, the intended storage time and the storage temperature. According to one embodiment, the stabilization composition is mixed with the sample in a ratio selected from 1:1000 to 1000:1, 1:100 to 100:1, 1: 75 to 75:1, 1:60 to 60:1, 1: 50 to 50:1, 1: 40 to 40:1, 1: 30 to 30:1, 1:25 to 25:1, 1:20 to 20:1, 1: 15 to 15:1, 1:10 to 10:1, 1:5 to 5:1, 1:3 to 3:1, 1:2 to 2:1 and 1:1. It is an advantage that already a small amount of the stabilization composition is sufficient to stabilize even difficult samples such as whole blood or blood products. E.g. as is shown in the examples, a ratio of 1:50 (e.g. 200μl stabilization composition mixed with 10ml blood) is sufficient to stabilize whole blood samples. Thus, according to one embodiment, the stabilization composition is contacted with the sample, which preferably is a body fluid, more preferred blood or a sample derived from blood, in a ratio selected from the following ranges 1:75 to 1:1, preferably 1:60 to 1:5, more preferred 1:50 to 1:10.

Suitable stabilization compositions which can be used for stabilizing samples, in particular blood samples, are also described in US 2011/0111410.

Samples that were stabilized as described above may be kept e.g. stored without refrigeration, preferably at room temperature, for a time period of at least one day, at least two days, at least three days, at least one day to three days, or selected from at least one day to seven days and/or at least one day to fourteen days (two weeks). Good stabilization effects were observed even at prolonged storage periods.

As is described above and as is demonstrated by the examples, the described stabilization of the sample allows for stabilizing the sample without refrigeration or freezing for a prolonged period of time period. Thus, the samples can be kept at room temperature or even at elevated temperatures e.g. up to 30 °C or up to 40 °C. According to one embodiment, a stabilization effect is achieved for at least two days, preferably at least three days; more preferred for a period of at least one day to six days, most preferred for at least one day to at least seven days at room temperature. Thus, as the samples were not compromised in particular when using the preferred combination of additives, even longer storage/shipping times are conceivable. However, usually, longer periods are not necessary, as the regular storage and e.g. shipping time to the laboratory, wherein the nucleic acid isolation and optionally cell analysis is performed, usually does not exceed 6 or 7 days, but usually is even completed after two or three days.

The stabilizing composition described above can be e.g. present in a device for collecting the sample, which preferably is a body fluid sample, or can be added to a respective collection device immediately prior to collection of the sample or can be added to the collection device preferably immediately after the sample was collected therein. Preferably, the collection device is a blood collection device. Preferably, said collection device is an evacuated collection container, such as a tube. It is also within the scope to add the formaldehyde releaser agent(s) and optionally, the further additive(s) separately to the sample. However, for the ease of handling, it is preferred that the one or more of the additives are provided in one stabilization composition. Furthermore, in an advantageous embodiment, a formaldehyde releaser agent and optionally the further additives such as an enzyme inhibitor, a metabolic inhibitor and/or a metal ion chelator are present in a collection device prior to adding the sample. This ensures that the sample is immediately stabilized upon contact with the stabilizing composition. The stabilizing composition is present in the container in an amount effective to provide the stabilization of the amount of sample to be collected, respectively comprised in said container.

As discussed above, the stabilization according to the present disclosure inter alia has the effect that cells contained in the sample and also the cell morphology can be substantially preserved in the state they have shown at the time the sample was obtained, respectively drawn. Furthermore, also nucleic acids, including extracellular nucleic acids comprised in the sample are efficiently stabilized.

As discussed above, even though using a formaldehyde releaser for stabilization is advantageous with respect to the achieved stabilization effect, a respective stabilization procedure causes significant problems with respect to the subsequent isolation of the nucleic acids. As is demonstrated in the present examples, standard methods that are suitable for isolating nucleic acids from various sample types such as e.g. phenol-chloroform based nucleic acid isolation procedures as well as nucleic acid isolation procedures that are even recommended in the prior art as being suitable for isolating nucleic acids from respectively stabilized samples are often ineffective in isolating nucleic acids with acceptable yield and purity. The present invention provides a solution to this problem by finding that the nucleic acid isolation can be greatly improved when using a cationic detergent during the sample preparation for nucleic acid isolation and in particular when using a cationic detergent for lysis of the sample.

The term "lysis" as used herein refers to the disruption, degradation and/or digestion of a sample or portion or fraction thereof. In a respective lysis step, biomolecules such as in particular nucleic acids can be released from cells or can be freed from other sample components such as e.g. proteins. Herein, we refer to a respective step to disrupt, degrade and/or digest a sample generally as lysis step, irrespective of whether biomolecules such as in particular nucleic acids are released from cells or whether the lysis is performed in order to release biomolecules such as nucleic acids e.g. from proteins or other substances comprised in the sample. Hence, the sample may comprise cells or may comprise no or only minor amounts of cells as is e.g. the case with blood plasma. The degradation and/or digestion of a respective cell-free or cell-depleted sample to release the nucleic acids is also referred to herein as lysis. Preferably, the nucleic acids are protected from degradation during lysis. The present invention is based on the finding that the use of a cationic detergent during lysis of the stabilized sample or portion or fraction thereof improves the nucleic acid isolation results.

Several options exist how the obtained stabilized sample can be further processed in order to isolate nucleic acids from the stabilized sample or portion or fraction thereof.

According to one embodiment, the stabilized sample is further processed prior to contact with the cationic detergent for initial lysis. According to one embodiment, cells contained in the stabilized sample are isolated and/or are analysed using immuno-mediated methods such as e.g. flow cytometry and non immuno-mediated approaches including but not limited to cell capture and/or cell sorting methods based on e.g. cell size or cell adhesion. Microscopic analyses are also feasible. In particular for the purpose of cell analysis, it is important that the stabilization composition and also the whole stabilization process does not initiate or promote the lysis of the cells. However, the inhibition of cell lysis due to the stabilization of the sample is also important to prevent a contamination of extracellular nucleic acids with intracellular nucleic acids if a sample is processed that comprises extracellular nucleic acids and cells as is the case e.g. with blood and urine. The formaldehyde releaser based technology described above and in the examples do not induce cell lysis but rather reduce cell lysis and accordingly, stabilize the cells comprised in the sample.

According to one embodiment, the nucleic acids are only isolated from a portion or fraction of the stabilized sample. Thus, prior to lysis involving the cationic detergent, a portion or fraction of the stabilized sample is obtained. According to one embodiment, said portion or fraction comprises or predominantly consists of cells. E.g. cells in general or a specific cell type may be isolated from the stabilized sample and the nucleic acids are subsequently isolated from the obtained cells. This embodiment is e.g. suitable if a cell-containing sample such as e.g. a whole blood sample is processed. Here, e.g. white blood cells can be isolated from the stabilized blood sample using appropriate means. Optionally, red blood cells may be lysed prior to collecting the white blood cells using an appropriate lysis buffer for red blood cells. Preferred embodiments are described below. Isolating cells from the stabilized sample is also advantageous when intending to analyse a specific cell type comprised within other cell types, e.g. a complex tissue sample and in particular is advantageous for the specific analysis of tumor cells. Besides allowing an analysis of the cells itself, e.g. the cell morphology, it is also possible to isolate the nucleic acids specifically from the respectively isolated cells using the teachings of the present invention. According to a further embodiment, said portion or fraction of the stabilized sample comprises or consists of a cell-free or cell-depleted portion or fraction of the stabilized sample. E.g. when processing a cell-containing stabilized sample such as e.g. a whole blood sample, cells can be removed from the stabilized sample using suitable means (e.g. by centrifugation or by binding the cells to appropriate cell adsorbing or cell binding surfaces) and the nucleic acids are isolated from the respectively obtained cell-depleted or depending on the removal process even cell-free portion or fraction of the original stabilized sample. This embodiment is particular useful if intending to isolate e.g. extracellular nucleic acids from a stabilized sample. Extracellular nucleic acids can be indicative of a health issue and their isolation and analysis is thus of high diagnostic value, e.g. in the field of cancer or other diseases. Furthermore, also fetal nucleic acids are found as extracellular nucleic acids in maternal blood. Furthermore, it is within the scope of the present invention to isolate nucleic acids according to the teachings of the present invention from different portions or fractions from the same stabilized original sample. E.g. the original stabilized sample can be fractionated in a cell containing portion and in a cell-depleted or even cell-free portion and nucleic acids can then be isolated separately from each of said separate portions if desired. Thereby, it is e.g. possible to isolate intracellular nucleic acids (from the cell-containing portion) separately from extracellular nucleic acids (from the cell-depleted or even cell-free portion). The respectively isolated nucleic acids can then be analysed as e.g. described herein. Furthermore, the cell containing portion or fraction of the stabilized sample can be additionally used to analyse the cells, e.g. their morphology. For this purpose the obtained cell containing portion or fraction of the stabilized sample may be further divided, wherein one portion is used for cell analysis and the other for nucleic acid isolation.

The stabilized sample or stabilized portion or fraction of the stabilized sample from which the nucleic acids shall be isolated, e.g. cells comprised in the stabilized sample, is contacted for lysis with at least one cationic detergent. For the ease of simplicity, subsequently we refer generally to the processing of the stabilized sample. However, our subsequent explanations also apply equally to the processing of a portion or fraction of the stabilized sample and accordingly, also these embodiments form part of the present disclosure. Specific embodiments are also described in detail.

The inventors found that the nucleic acid isolation from a sample that was stabilized using a formaldehyde releaser or from a portion or fraction thereof can be severely improved, if the lysis of the sample involves the use of a cationic detergent. As is shown by the examples, the yield and purity can be significantly improved. Suitable and preferred examples of cationic detergents will be described below.

According to one embodiment, during lysis of the stabilized sample or portion or fraction thereof at least one cationic detergent having the following formula (1) is used:

$$Y^+R_1R_2R_3R_4X^- \qquad (1)$$

wherein

Y is nitrogen or phosphorus;

R1, R2, R3 and R4 idependently are selected from a branched or unbranched C1-C20 alkyl residue, a C3 to C6 alkylene residue, a C3 to C6 alkinyl residue and/or a C6-C26 aralkyl residue and wherein preferably at least one of R1, R2, R3 or R4 is a C6 to C20 alkyl residue and even more preferred is at least a C10 alkyl residue;

X$^-$ is the anion of an anorganic or organic mono- or polybasic acid.

Examples of respective cationic detergents include but are not limited to quarternary ammonium salts, amines with amide linkage, polyoxyethylene alkyl and alicyclic amines, N,N,N',N'tetrakis substituted ethylenediamines, 2-alkyl 1-hydroxyethyl 2 imidazoline ethoxylated amines and alkyl ammonium salts.

According to one embodiment, the cationic detergent is provided in a lysis composition comprising

a) a cationic compound of the general formula (1):

$$Y^+R_1R_2R_3R_4X^- \qquad (1)$$

wherein Y represents nitrogen or phosphorus, preferably nitrogen

$R_1R_2R_3$ and $R_4$ independently, represent a branched or unbranched $C_1$-$C_{20}$-alkyl group, a $C_6$-$C_{20}$-aryl group and/or a $C_6$-$C_{26}$ aralkyl group;

X$^-$ represents an anion of an inorganic or organic, mono- or polybasic acid; and

b) at least one proton donor, wherein the proton donor is preferably present in the composition in a concentration of above 50 mM to saturation and wherein the proton donor is preferably selected from the group consisting of saturated aliphatic monocarboxylic acids, unsaturated alkenyl-carboxylic acids, saturated and/or unsaturated aliphatic $C_2$-$C_6$-dicarboxylic acids, aliphatic hydroxyl-di- and tricarboxylic acids, aliphatic ketocarboxylic acids, amino acids or the inorganic acids or the salts thereof, on their own or in combination.

Using a respective cationic detergent is particularly suitable when intending to isolate RNA of high quality. Preferably, $R_1$ denotes a higher alkyl group with 12, 14 or 16 carbon atoms and $R_2$, $R_3$ and $R_4$ each represent a methyl group. Preferably, the anion X$^-$ represents an anion of hydrohalic acids or anions of mono- or dibasic organic acids, most preferred the anion X$^-$ is selected from the group consisting of bromide, chloride, phosphate, sulphate, formate, acetate, propionate, oxalate, malonate, succinate or citrate. Preferably, the proton donor is selected from the group consisting of saturated aliphatic monocarboxylic acids, unsaturated alkenyl-carboxylic acids, saturated and/or unsaturated aliphatic $C_2$-$C_6$ -dicarboxylic acids, aliphatic ketocarboxylic acids, amino acids or the inorganic acids or the salts thereof, and combinations thereof. Preferably, the aliphatic monocarboxylic acid comprises a $C_1$-$C_6$-alkyl-carboxylic acid selected from the group consisting of acetic acid, propionic acid, n-butyric acid, n-valeric acid, isovaleric acid, ethyl-methyl-acetic acid (2-methyl-butyric acid), 2,2-dimethylpropionic acid (pivalic acid), n-hexanoic acid, n-octanoic acid, n-decanoic acid or n-dodecanoic acid (lauric acid) or mixtures thereof. Preferably, the aliphatic alkenyl-carboxylic acid is selected from the group consisting of acrylic acid (propenoic acid), methacrylic acid, crotonic acid, isocrotonic acid or vinylacetic acid or mixtures thereof. Preferably, the saturated aliphatic $C_2$-$C_6$-dicarboxylic acid is selected from the group consisting of oxalic acid, malonic acid, succinic acid, glutaric acid or adipic acid or mixtures thereof. Most preferred, the aliphatic dicarboxylic acid is oxalic acid or succinic acid or mixtures thereof. Preferably, the aliphatic hydroxy-di- and -tricarboxylic acids are selected from the group consisting of tartronic acid, D-(+), L-(-) or DL-malic acid, (2R, 3R)-(+)-tartaric acid, (2S, 3S)-(-)-tartaric acid, meso-tartaric acid and citric acid or mixtures thereof. Most preferred, the unsaturated dicarboxylic acid is maleic and/or fumaric acid or mixtures thereof. Preferably, the unsaturated tricarboxylic acid is aconitic acid. Preferably, the aliphatic ketodicarboxylic acids are mesoxalic acid or oxaloacetic acid, or mixtures thereof. Preferably, the amino acids are selected from the group consisting of aminoacetic acid (glycine), alpha -aminopropionic acid (alanine), alpha -amino-iso-valeric acid (valine), alpha -amino-iso-caproic acid (leucine) and alpha -amino- beta -methylvaleric acid (isoleucine), or mixtures thereof.

Suitable lysis compositions comprising a respective cationic detergent according to formula 1 and a proton donor are also described in detail e.g. in US 7,270,953. Therein, they are in particular described as stabilising agents. Here, the inventors now found that these stabilizing agents can be advantageously used to lyse a sample or portion or fraction thereof that was stabilized by the use of at least one formaldehyde releaser.

According to one embodiment, a detergent is used for lysis which comprises under the used conditions a charged quarternary ammonium cation as polar head group and thus is or becomes cationic under the used lysis conditions. Thus, according to one embodiment of the present invention a detergent is used which is cationic at the used lysis conditions. Accordingly, also an originally non-ionic detergent can be used if it is or becomes cationic during lysis. Thus, besides cationic detergents comprising a permanently charged head group also an originally ternary amine can be used as cationic detergent if provided in an acidic environment whereby the ternary amine incorporates a proton and become positively charged. According to one embodiment, an amino surfactant having the following formula 2 is used as detergent which is or becomes cationic under the used lysis conditions and thereby functions as cationic detergent:

$$R1R2R3N(O)x \qquad (2)$$

wherein,

R1 and R2 each independently is H, C1-C20 alkyl residue, C6-C26 aryl residue or C6-C26 aralkyl residue, preferably H, C1-C6 alkyl residue, C6-C12 aryl residue or C6-C12 aralkyl residue,

R3 is C1- C20 alkyl group, C6-C26 aryl residue or C6-C26 aralkyl residue,

X is an integer of 0 and 1.

According to one embodiment, x is 1 and R1 and R2 each independently is C1 -C6 alkyl, and R3 is C1 -C20 alkyl. According to a preferred embodiment, x is 0. According to one embodiment, said amino surfactant is selected from the group consisting of dodecylamine, N-methyldodecylamine, N, N-dimethyldodecylamine, N, N- dimethyldodecylamine N oxide and 4-tetradecylaniline. Preferably, said amino surfactant is comprised in a composition which additionally comprises at least one proton donor, preferably an acid or acid salt to render a quarternary ammonium cation. According to one embodiment, the composition comprises at least one acid salt selected from the group consisting of maleic acid, tartaric acid, citric acid, oxalic acid, carboxylic acids and mineral acids. The total concentration of said acid salt in the composition may range from 0.01 M to 1M. Said composition can be added to the sample to induce lysis. However, the proton donor may also be comprised in the stabilized sample or it may be provided by a composition such as a solution which is added separately from the amino surfactant to the stabilized sample.

According to a preferred embodiment, a cationic detergent is used which comprises a permanently charged quaternary ammonium cation as polar head group. As is shown in the examples, respective cationic detergents are suitable to improve the nucleic acid isolation from samples that were stabilized using a formaledehyde releaser. Preferably, the cationic detergent is an alkyltrimethylammonium salt. Preferably, the cationic detergent comprises ammonium bromide or ammonium chloride. Most preferably, the cationic detergent is selected from the group consisting of cetyl trimethyl ammonium bromide (CTAB), tetra decyl trimethyl ammonium bromide (TTAB) and dodecyl trimethyl ammonium bromide (DTRB) or the corresponding compounds comprising a chloride instead of the bromide. As is shown by the examples, a respective cationic detergent can be advantageously used for lysis, wherein the nucleic acid isolation results are considerably improved.

Further cationic detergents include but are not limited to didecyldimethylammoniumchlorid, benzalkoniumchloride, n-dodecyl trimethyl ammonium bromide (DTAB), trimethyl-tetradecylammoniumbromid, N, N' dimethyldodecylamine-N-oxide ctenidine dihydrochloride; alkyltrimethylammonium, salts hexadecyl trimethyl ammonium bromide, dimethyldioctadecylammonium chloride, dioctadecyldimethylammonium bromide (DODAB), hexadecyltrimethylammonium bromide (HTAB), cetylpyridinium chloride, dimethyl dioctadecyl ammonium bromide, alkyl hydroxyethyl dimethyl ammonium chloride and distearyl dimethyl ammonium chloride.

According to one embodiment, the method according to the present invention comprises the following steps:

a. obtaining the stabilized sample or a portion or fraction thereof;

b. contacting the stabilized sample or a portion or fraction thereof with at least one cationic detergent and optionally further lysis agents for lysis and thereby providing a lysed sample; and

c. isolating nucleic acids.

In a specific embodiment, which is in particular advantageous when processing stabilized blood samples and in particular when intending to isolate nucleic acids, in particular RNA from blood cells, in particular white blood cells, the method according to the present invention comprises the following steps:

a. obtaining cells, which preferably are white blood cells, from the stabilized sample, wherein said cells constitute a portion or fraction of the stabilized sample;

b. contacting the cells with at least one cationic detergent and optionally further lysis agents for lysis and providing a lysed sample; and

c. isolating nucleic acids.

The composition that is formed in step b. when contacting the stabilized sample or portion or fraction thereof with the at least one cationic detergent for lysis provides a lysed sample. Preferably, the composition comprising the stabilized sample or portion or fraction of the stabilized sample and the at least one cationic detergent is incubated to provide the lysed sample and to ensure that the nucleic acids are prepared for isolation. Furthermore, optionally additional lysis agents can be added to assist and/or speed up the lysis of the sample such as e.g. proteolytic enzymes, chaotropic agents or other detergents. They can be added at the same time as the cationic detergent, optionally in one composition or solution, or can be added sequentially. However, as is shown by the examples the addition of respective additional lysis reagents is not required and according to one embodiment no further lysis agents are added in step b.. Furthermore, it is also possible to perform intermediate steps between the addition of the cationic detergent and the addition of the further lysis agents. Suitable examples are described below.

The concentration of the cationic detergent in the lysis composition that is obtained when contacting the stabilized sample or portion or fraction thereof with the cationic detergent and the optional further lysis agents to assist the lysis in step b. can be at least 0.25% (w/v), at least 0.5% (w/v), at least 0.75% (w/v), at least 1% (w/v), at least 1.25% (w/v), at least 1.5% (w/v), at least 1.75% (w/v), at least 2.5% (w/v) or at least 2.75% (w/v). Suitable concentration ranges for the cationic detergent in the respective lysis composition that is obtained in step b. after contacting the cationic detergent and the optional additional lysis agents with the stabilized sample or portion or fraction thereof include but are not limited to 0.25% (w/v) to 30% (w/v), 0.25% (w/v) to 25% (w/v), 0.25% (w/v) to 20% (w/v), 0.5% (w/v) to 15% (w/v), 0.75% (w/v) to 12.5% (w/v), 1% (w/v) to 10% (w/v), 1.25% (w/v) to 7.5% (w/v) and 1.5% (w/v) to 5% (w/v).

According to one embodiment, step b. comprises incubating the lysis composition that is obtained when contacting the stabilized sample or portion or fraction thereof with the cationic detergent and the optional additional lysis agents for a time sufficient to provide a lysed sample. Said incubation may occur for at least 15min, at least 30min, at least 45min, at least 1 h, at least 1.5h, at least 2h, at least 2.5h, at least 3h, at least 3.5h, at least 4h, at least 4.5h, at least 5h, at least 5.5h or at least 6h. Longer incubation times generally result in an increase of nucleic acid yield. The required incubation time also depends on the type of the sample, the used incubation temperature and whether further lysis supporting agents are added or not.

According to one embodiment, the initial lysis in step b. does not involve a step wherein the lysis composition that is obtained when contacting the stabilized sample or portion or fraction thereof with the cationic detergent and the optional additional lysis agents is heated to a temperature of 85°C or above. According to certain embodiments, said lysis composition may be heated though to a temperature that lies in a range of 30°C to 70°C, which is e.g. suitable if a proteolytic enzyme such as proteinase K is used in step b. as additional lysis agent. However, according to one embodiment, the lysis in step b. does not involve a step wherein the lysis composition that is obtained when contacting the stabilized sample or portion or fraction thereof with the cationic detergent and the optional additional lysis agents is heated to a temperature of 75°C or above, 65°C or above, 60°C or above, 55°C or above, 50°C or above, 45°C or above, 40°C or above, 35°C or above or 30°C or above. Preferably, the initial lysis in step b. is carried out at room temperature.

According to one embodiment, a nucleic acid containing portion is obtained from the lysed sample and said nucleic acid containing portion is subjected to the nucleic acid isolation step c.. E.g., said nucleic acid containing portion can be obtained by sedimentation, which preferably is assisted by centrifugation. E.g. the nucleic acids may form complexes with the at least one cationic detergent that is used in step b. during lysis of the stabilized sample or portion or fraction thereof. This is e.g. the case when using a cationic detergent of formula 1 or a detergent of formula 2 (if rendered cationic due to the addition or presence of a proton donor). Said complexes comprise the nucleic acid to be isolated. Said complexes can be obtained from the sample e.g. by sedimentation or filtration. Thereby, usually other sample components such as proteins and cell debris are also obtained together with the nucleic acid containing complexes. Preferably, the nucleic acid containing complexes are obtained in form of a pellet. A respective pellet can be e.g. obtained by centrifuging the lysed sample and discarding the supernatant thereby obtaining a nucleic acid containing portion from the lysed sample which comprises the complexes comprising the nucleic acids and the cationic detergents. Depending on the type of sample to be processed, the respective nucleic acid containing pellet that is obtained from the lysed sample usually also comprises further sample components such as proteins and/or cell debris. This is in particular the case when processing complex samples such as e.g. whole blood or samples derived from blood such as e.g. white blood cells, serum or plasma. Obtaining a nucleic acid containing portion, e.g. the complexes in form of a pellet, has the advantage that the subsequent nucleic acid isolation can be performed in smaller volumina what is cost-efficient as less reagents are necessary for nucleic acid isolation. The generation of a pellet leads also to higher sensitivities, as larger sample volumes can be concentrated and processed. This also provides an important advantage when using automated methods for nucleic acid isolation because many automated systems are limited with respect to the volume they can process.

The lysed sample and/or the nucleic acid containing portion of the lysed sample may be frozen and stored prior to isolating the nucleic acids in step c..

After lysis according to the present invention involving the use of a cationic detergent, the nucleic acids are isolated. Said specific lysis renders the nucleic acids contained in the stabilized sample or portion or fraction thereof accessible. Therefore, many nucleic acid isolation methods can now be used due to the specific lysis of the stabilized sample that is performed according to the present invention. Suitable nucleic acid isolation methods are known in the prior art and include but are not limited to extraction, solid-phase extraction, silica-based purification methods, nucleic acid isolation procedures using a chaotropic agent and/or at least one alcohol and a nucleic acid binding solid phase, magnetic particle-based purification, anion-exchange chromatography (using anion-exchange surfaces), precipitation, chromatin immunoprecipitation and combinations thereof. Preferably, the nucleic acids are isolated using an automated system. Preferably, the nucleic acids are isolated from a plurality of samples. The plurality of samples can be processed batchwise.

As described, the nucleic acids can be isolated in step c. from the lysed sample or from an aliquot thereof. Furthermore, the nucleic acids can be isolated from a nucleic acid containing portion that is obtained from the lysed sample (e.g. in form of nucleic acid containing complexes or in form of a nucleic acid containing supernatant). In this case, said portion is obtained prior to performing the nucleic acid isolation in step c..

According to one embodiment, in step c. of the method according to the present invention the lysed sample or the nucleic acid containing portion obtained from the lysed sample is contacted with one or more additional lysing agents thereby providing a lysis mixture. Thus, the lysis mixture is obtained by further digesting, homogenising and/or breaking up the lysed sample, respectively the nucleic acid containing portion thereof. Thereby, the comprised nucleic acids are efficiently released and accordingly available, respectively accessible for isolation. This further digestion is done under conditions which preserve the comprised nucleic acids from degradation. Examples of respective lysing agents include but are not limited to proteolytic enzymes, detergents and chaotropic agents. Details are described below. Such processing step is optional, if the lysis in step b., which can be assisted by adding lysis agents as described above, renders a respective second digestion step obsolete.

Optionally, the method may comprise further treatment steps in order to prepare the lysed sample for nucleic acid preparation. Examples of respective additional steps are described in further detail below.

If the lysed sample or the nucleic acid containing portion obtained from the lysed sample comprises solid matter, e.g. the complexes and optional other sample components, which are preferably obtained in form of a pellet, it can be resuspended, thereby obtaining a resuspended sample. Said resuspended sample comprises the nucleic acid to be isolated, the cationic detergent and optionally further sample components such as e.g. proteins and/or cell debris that were collected together with the complexes. Furthermore, optionally further additives can be added either before, during or after resuspension such as e.g. a chaotropic agent and/or a protein degrading compound to assist the further lysis. Herein, we refer to the resuspended lysed sample as well as to the nucleic acid containing portion obtained from the lysed sample (e.g. the nucleic acid containing complexes that were obtained from the lysed sample) including optional further sample components and/or optional additives that were added before, during and/or after resuspension as "resuspended sample". The resuspended sample may comprise at least one chaotropic agent. Preferably, the chaotropic agent is added as separate additive to provide a resuspended sample comprising a chaotropic agent. Preferably, the chaotropic agent is added to the optionally already resuspended complexes in form of an aqueous solution as is described below in order to generate a resuspended sample comprising *inter alia* the nucleic acids and the chaotropic agent. The chaotropic agent protects the nucleic acids, in particular RNA, from degradation, thereby increasing the quality of the isolated nucleic acids. Furthermore, it supports the further lysis.

According to one embodiment, a resuspension solution is added to the lysed sample wherein said resuspension solution comprises a salt, preferably a non-chaotropic salt. As salt, several salts can be used including but not being limited to ammonium salts and alkali metal salts, preferably ammoinium acetate, ammonium sulphate, KCl or NaCl.

Preferably, an ammonium salt is used. A chelating agent may be comprised in the resuspension solution. Furthermore, a chaotropic agent can be added before the complexes were resuspended and accordingly, before the resuspension solution was added. This order is beneficial when processing e.g. white blood cells obtained from a stabilized blood sample. According to one embodiment, the resuspension solution comprises the chelating agent in a concentration of at least 1 mM, at least 5mM, preferably at least 7.5mM, more preferred at least 10mM, at least 15mM or at least 20mM. Preferably, the concentration range is selected from 1mM to 150mM, 5mM to 100mM, 5mM to 75mM, 7.5mM to 65mM, 7.5mM to 50mM and 10mM to 30mM. In some embodiments to use a lower to medium concentration of the chelating agent e.g. in a range of 1 mM to 50mM, preferably 5mM to 30mM or 7.5mM to 20mM. The chelating agent may also be added separately from the resuspension solution, e.g. in liquid or solid form.

According to one embodiment, the chelating agent is added in a concentration so that the resuspended sample comprises the chelating agent in a concentration of at least 0.5mM, at least 2.5mM, preferably at least 3.5mM, more preferred at least 5mM, at least 7.5mM or at least 10mM. Preferably, the chelating agent is added in a concentration so that the resuspended sample comprises the chelating agent in a concentration selected from 0.5mM to 100mM, 2.5mM to 75mM, 2.5mM to 60mM, 3.5mM to 50mM, 3.5mM to 30mM, 3.5mM to 25mM, 3.5mM to 20mM, 5mM to 15mM and 5mM to 10mM.

Chelating agents according to the present invention include, but are not limited to diethylenetriaminepentaacetic acid (DTPA), ethylenedinitrilotetraacetic acid (EDTA), ethylene glycol tetraacetic acid (EGTA) and N,N-bis(carboxymethyl)glycine (NTA). According to a preferred embodiment, EDTA is used. As used herein, the term "EDTA" indicates *inter alia* the EDTA portion of an EDTA compound such as, for example, $K_2EDTA$, $K_3EDTA$ or $Na_2EDTA$.

As described above, in nucleic acid isolation step c., lysis of the sample can be continued or completed to improve the nucleic acid isolation. Thus, additional lysis agents can be added. E.g. any chaotropic agent can be used to assist the further lysis that causes disorder in a protein or nucleic acid by, for example, but not limited to altering the secondary, tertiary or quaternary structure of a protein or a nucleic acid while leaving the primary structure intact. Preferably, a chaotropic salt is used. Preferred chaotropic agents include but are not limited to guanidinium hydrochloride, guanidinium thiocyanate, guanidinium isothiocyanate, sodium thiocyanate, sodium iodide, sodium perchlorate, sodium trichloroacetate, sodium trifluroacetate, urea and the like. Preferably, the chaotropic agent is GTC or GITC or an equally strong chaotropic agent. Respective strong chaotropic agents are very efficient in protecting the nucleic acid, in particular RNA, from degradation. According to one embodiment, the resuspended sample comprises a chaotropic agent in a concentration selected from the group consisting of 0.1 M to saturation, 0.5M to 5M, 0,5M to 4M, 0,5M to 3M, 0.75M to 2.5M, most preferred at least 1M. The at least one chaotropic agent may be added to assist the lysis procedure in form of a separate composition, e.g. a separate solution that is added to the lysed sample or a nucleic acid containing portion thereof to provide a lysis mixture. Said separate solution preferably comprises a chaotropic salt, e.g. a guanidinium salt, a buffer and/or a chelating agent. Preferably, said buffer comprises sodium citrate.

The pH value of the lysis mixture preferably lies in a range that is selected from 3 to 10, 4 to 10, 5 to 10, 5.5 to 9.5, 6 to 9, 6.5 to 8.5 and preferably 7 to 8. In a pH range of 5 to 8.5, pellets obtained from the lysis of whole blood or selected cells such as white blood cells were particularly well resuspended. Therefore, according to one embodiment, a resuspension solution is added to the complexes, preferably the pellet comprising the cationic detergent and the nucleic acids which has a pH value that achieves, with the added amount, a pH value in the resuspended sample that lies in the above defined pH range. According to one embodiment, the pH value of the resuspension solution lies in a pH range that is selected from 5 to 10.5, 5.5 to 10, 5.7 to 10, 6 to 9.7, 6.3 to 9.5, 6 to 9 and 6 to 8.5. Preferably, the pH range lies with a range of 5.7 to 10, more preferred 6 to 9. Here, the achieved nucleic acid yield was optimal.

According to one embodiment, a protein degrading compound is incorporated during lysis in step b. and/or c. to assist the lysis. According to a preferred embodiment, the protein-degrading compound is a proteolytic enzyme. A proteolytic enzyme refers to an enzyme that catalyzes the cleavage of peptide bounds, for example in proteins, polypeptides, oligopeptides and peptides. Exemplary proteolytic enzymes include but are not limited to proteinases and proteases in particular subtilisins, subtilases, alkaline serine proteases and the like. Subtilases are a family of serine proteases, i.e. enzymes with a serine residue in the active side. Subtilisins are bacterial serine protease that has broad substrate specificities. Subtilisins are relatively resistant to denaturation by chaotropic agents, such as urea and guanidine hydrochloride and anionic detergents such as sodium dodecyl sulfate (SDS). Exemplary subtilisins include but are not limited to proteinase K, proteinase R, proteinase T, subtilisin, subtilisin A, QIAGEN Protease and the like. Discussions of subtilases, subtilisins, proteinase K and other proteases may be found, among other places in Genov et al., Int. J. Peptide Protein Res. 45: 391-400, 1995. Preferably, the proteolytic enzyme is proteinase K. In non-limiting aspects, the proteolytic enzyme is comprised in the resuspended sample in a concentration between about 0.05 mg/ml to about 10 mg/ml. In other embodiments the range can be between from about 0.1 mg/ml to about 5 mg/ml, or between about 0.2 mg/ml to about 1.0 mg/ml.

In order to efficiently prepare the stabilized sample or portion or fraction thereof for the nucleic acid isolation it is preferred to thoroughly digest the sample or portion or fraction thereof prior to isolating the nucleic acids. Here, different options exist that may be used in conjunction with the present invention. Some non-limiting options are subsequently described.

According to one embodiment, a proteolytic enzyme is used during lysis in step b. and/or preferably in step c. The sample comprising the proteolytic enzyme is incubated under conditions that support the digestion, preferably under heating and agitation for at least 3min, preferably at least 5min, more preferred at least 10min.

According to one embodiment, the conditions that allow the digestion of the sample in the presence of a proteolytic enzyme comprise one or more of the following

a) heating,
b) agitation,
c) the presence of salts,
d) a pH value of between 6 to 9 and/or
e) an incubation period of at least 3 min, preferably at least 5min, most preferred for at least 10 min.

Preferably, said incubation step is performed under heating. Preferably, the sample comprising the proteolytic enzyme, e.g. the resuspended sample obtained as described above, is heated at least to a temperature of 15° C, at least 25° C, at least 35° C, at least 40° C, or at least 50° C and preferably is heated to a temperature of at least 55° C during incubation. Using respective higher temperatures during incubation is in particularly favourable if a proteolytic enzyme such as proteinase K is used as protein-degrading compound that shows its optimal, respective highest activity at higher temperatures. Under such conditions, the digestion is promoted. Of course, a temperature should be used wherein the proteolytic enzyme is active. Furthermore, it is preferred that the said incubation step is performed while agitating the resuspended sample. Non-limiting examples of agitation include shaking, stirring, mixing, or vibrating. In certain aspects, agitation comprises shaking. The shaking can be one, two, or three dimensional shaking. A variety of shaking or agitating devices can be used. Non-limiting examples include the Thermomixer (Eppendorf), TurboMix (Scientific Industries), Mo Bio Vortex Adapter (Mo Bio Laboratories), Microtube holder vortex adapter (Troemner), and the Microtube foam rack vortex attachment (Scientific Industries). Agitating can be performed for example in a mixer with at least 50rpm, at least 100rpm, at least 200rpm, at least 500rpm or at least 1,400rpm. Preferably, heating and agitation is simultaneously performed, for example by using a thermomixer or an equivalent apparatus that allows simultaneous heating and agitation. When using at least one proteolytic enzyme as protein-degrading compound, incubation conditions are used that ensure that said enzyme works efficiently and is catalytically active. The conditions depend on the proteolytic enzyme used and are known, respectively determinable by the skilled person. Preferably, the incubation is performed in the presence of salts and/or ions that promote and/or maintain the activity of the proteolytic enzyme. Suitable salts include but are not limited to NaCl, KCl, $MgCl_2$, or $CaCl_2$ or chaotropic agents such as chaotropic salts. The above described conditions are particularly favourable when using a proteolytic enzyme as protein-degrading compound and said conditions promote the digestion.

Furthermore, preferably, as described above at least one chaotropic agent is included in the lysis procedure of step b. and/or c., preferably at least in step c. to preserve the integrity of the comprised nucleic acids, in particular the RNA. Thus, the digestion may be performed in the presence of at least one chaotropic agent, preferably a chaotropic salt. For this purpose a digestion solution can be added which comprises at least one chaotropic agent. Said digestion solution may also comprise additional compounds such as e.g. detergents and salts that promote the digestion and/or preserve the comprised nucleic acid. Any chaotropic agent can be used for that purpose that causes disorder in a protein or nucleic acid by, for example, but not limited to altering the secondary, tertiary or quaternary structure of a protein or a nucleic acid while leaving the primary structure intact. Preferred chaotropic agents that can be used during incubation with the at least one protein-degrading compound are chaotropic salts which include but are not limited to guanidinium hydrochloride, guanidinium thiocyanate, guanidinium isothiocyanate, sodium thiocyanate, sodium iodide, sodium perchlorate, sodium trichloroacetate, sodium trifluroacetate, urea and the like.

The incubation with the at least one protein-degrading compound is usually performed at a pH value that does not lead to a degradation of the comprised nucleic acid. Furthermore, when using a proteolytic enzyme as protein-degrading compound, a pH value should be used wherein the proteolytic enzyme is active. Preferably, the incubation with the at least one protein-degrading compound for digesting the resuspended sample is performed at a pH between 4.3 to 9, 6 to 8 and, preferably, is performed at a neutral pH value.

After optionally, but preferably digesting the resuspended sample as described above as initial step of the nucleic acid isolation procedure, the nucleic acid can be e.g. bound to a solid phase and the nucleic acid can be optionally eluted therefrom. Preferably, the nucleic acid isolation involves the use of at least one chaotropic salt and/or alcohol. Preferred embodiments are described below.

As solid phase, any material that is capable of binding nucleic acids that are present in or are released from a sample can be used and include a variety of materials that are capable of binding nucleic acids under suitable conditions. Exemplary solid phases that can be used in conjunction with the present invention include, but are not limited to, compounds comprising silica, including but not limited to, silica particles, silica fibres, glass fibres, silicon dioxide, diatomaceous earth, glass, alkylsilica, aluminum silicate, and borosilicate; nitrocellulose; diazotized paper; hydroxyapatite (also referred to as hydroxyl apatite); nylon; metal oxides; zirconia; alumina; polymeric supports, diethylaminoethyl- and triethylaminoethyl-derivatized supports, hydrophobic chromatography resins (such as phenyl- or octyl Sepharose) and the like. The term solid phase is not intended to imply any limitation regarding its form or design. Thus, the term solid phase encompasses appropriate materials that are porous or non-porous; permeable or impermeable; including but not limited to membranes, filters, sheets, particles, magnetic particles, beads, gels, powders, fibers, and the like. According to one embodiment, the surface of the solid phase such as e.g. the silica solid phase is not modified and is, e.g., not modified with functional groups.

According to a preferred embodiment, a solid phase comprising silica is used. Silica based nucleic acid isolation methods are broadly used in the prior art. The solid phase comprising silica may e.g. have the form of a filter, fibers, membrane or particles. In particular preferred is the use of silica particles that can be used in form of beads and which preferably have a particle size of about 0.02 to 30 $\mu$m, more preferred 0.05 to 15 $\mu$m and most preferred of 0.1 to 10 $\mu$m. To ease the processing of the nucleic acid binding solid phase, preferably magnetic silica particles are used. Magnetic particles respond to a magnetic field. The magnetic silica particles may e.g. be ferrimagnetic, ferromagnetic, paramagnetic or superparamagnetic. Suitable magnetic silica particles are for example described in WO 01/71732, WO 2004/003231 and WO 2003/004150. Other magnetic silica particles are also known from the prior art and are e.g. described in WO 98/31840, WO 98/31461, EP 1 260 595, WO 96/41811 and EP 0 343 934 and also include for example magnetic silica glass particles.

According to one embodiment, binding of the nucleic acids to the solid phase is performed under conditions having one or more, preferably at least two, preferably at least three of the following characteristics:

a) binding is performed in the presence of at least one chaotropic agent,
b) binding is performed in the presence of at least one alcohol,
c) binding is performed in the presence of at least one detergent,
d) binding is performed under conditions that promote binding of the nucleic acids, in particular the RNA, and/or
e) binding is performed under conditions that promote binding of small nucleic acids, in particular small RNA species.

According to one embodiment, the binding of the nucleic acids to the solid phase is performed in the presence of at least one chaotropic agent, preferably a chaotropic salt and/or in the presence of at least one alcohol. Also a mixture of chaotropic agents can be used. The concentration of the chaotropic agent or mixture of chaotropic agents that are used during binding may lie in a range of 0.05M up to the saturation limit. Preferred concentration ranges lie, depending on the chaotropic agent used, within 0.1M to 7M, 1M to 7M, 1.5M to 6M and 2M to 4M. Suitable chaotropic agents are in particular chaotropic salts and include but are not limited to guanidinium hydrochloride, guanidinium thiocyanate, guanidinium isothiocyanate, sodium thiocyanate, sodium iodide, sodium perchlorate, sodium trichloroacetate, sodium trifluoroacetate, urea and the like and in particular preferred are guanidinium hydrochloride, guanidinium thiocyanate and guanidinium isothiocyanate. The chaotropic agent that is present during binding may originate from the lysis procedure or may be added separately to establish the binding conditions. Furthermore, it is also within the scope of the present invention that an additional chaotropic agent is added for binding.

As alcohol that can be used to promote binding, it is preferred to use short chained branched or unbranched alcohols with preferably one to 5 carbon atoms. Examples are methanol, ethanol, propanol, isopropanol and butanol. Also mixtures of alcohols can be used. The alcohol is preferably selected from isopropanol and ethanol, particularly well suitable is isopropanol when isolating RNA as target nucleic acid. Preferably, the method according to the present invention does not involve the use of phenol and/or chloroform.

The alcohol may be comprised in the binding mixture in a concentration of 10% v/v to 90% v/v, in particular 15% v/v to 80% v/v, 20% to 80% v/v. The binding mixture in particular comprises the resuspended sample and the solid phase and may optionally comprise further agents that were added to establish, respectively improve the binding conditions. For isolating total RNA which also comprises small RNA, it is beneficial to use an alcohol concentration of $\geq$ 30% v/v, preferably $\geq$ 40% v/v to $\leq$ 90% v/v, more preferred $\geq$ 50% v/v to $\leq$ 90% v/v during binding and thus in the binding mixture. Respective higher concentrations of alcohol improve the binding and thus the isolation of short nucleic acids (usually having a size of 500nt or less), in particular small RNA species. Most preferred is an alcohol concentration of $\geq$ 40% v/v to $\leq$ 90% v/v during binding when intending to isolate RNA which includes small RNA. These concentrations work particularly well if the chaotropic agent(s) is/are present in higher concentrations and when binding the nucleic acids to a silica surface.

Thus, according to one embodiment, the nucleic acid isolation is performed using binding conditions having one or more of the following characteristics to bind the nucleic acids to a solid phase:

a) an alcohol concentration is used that is selected from the group consisting of 10% v/v to 90% v/v, 15%v/v to 90% v/v, 20% v/v to 85%v/v, 30%v/v to 80%v/v, 40% v/v to 85% v/v, 40%v/v to 80%, 40% v/v to 70%,

b) a concentration of one or more chaotropic agents is used that is selected from the group consisting of 0.05M up to the saturation limit, 0.1M to 6M and 1M to 4M, and/or

c) an alcohol concentration of at least 30% v/v and at least one chaotropic agent is used for binding RNA, including small RNAs to the solid phase.

To establish respective binding conditions, a binding solution which comprises e.g. the alcohol and/or the chaotropic agent can be added to the lysis mixture.

Optionally, one or more detergents can be added to the binding mixture to promote binding of the nucleic acid to the solid phase. Preferably, at least one ionic and/or at least one non-ionic detergent is added. Preferably, a non-ionic detergent is used in a concentration of at least 0.1 %. Said detergent can be added, e.g., together with the binding solution or can be provided by the resuspended sample and/or the digestion solution if a respective digestion solution is added to promote the digestion, respectively lysis of the sample.

Furthermore, a buffer can be used for binding, respectively can be incorporated in the binding solution. Non-limited examples are biological buffers which include but are not limited to HEPES, MES, MOPS, TRIS, BIS-TRIS Propane and others. Preferably, a Tris buffer is used in the binding solution.

Therefore, according to one embodiment, the nucleic acid isolation comprises the addition of a binding solution which comprises at least one alcohol and/or at least one chaotropic agent and optionally a biological buffer, preferably Tris, in order to establish the binding conditions that allow to bind the nucleic acid that are comprised in the resuspended sample to the solid phase. Optionally, the binding solution additionally comprises a detergent as is described above. However, the components can also be added separately to establish suitable binding conditions in the binding mixture. Preferably, the binding solution pH is in a range that includes 7. According to one embodiment, the pH of the binding solution is in the range from pH 6 to 9, preferably 6.5 to 8.5; most preferred the binding solution has a pH of 7 to 8.

According to one embodiment, the binding solution that is added to establish the binding conditions comprises or consists of alcohol.

According to one embodiment, one or more washing steps are performed in isolation step c. in order to further purify the isolated nucleic acids. According to one embodiment, one or more washing steps are performed while the nucleic acid is bound to the solid phase. For this purpose common washing solutions may be used. According to one embodiment, the solution used for washing comprises at least one chaotropic agent, at least one alcohol, at least one detergent and/or at least one buffering component. Chaotropic agents that can be used in the washing solutions include but are not limited to guanidinium hydrochloride, guanidinium thiocyanate, guanidinium isothiocyanate and sodium iodide. Furthermore, chaotropic salts can be used which comprise a chaotropic anion selected form the group consisting of trichloroacetate, perchlorate and trifluoroacetate. Examples of respective chaotropic salts are alkali salts like sodium perchlorate, sodium trichloroacetate and sodium trifluoroacetate. As alcohol, short chained branched or unbranched alcohols with preferably one to 5 carbon atoms can be used for washing, respectively in the washing solution. Examples are methanol, ethanol, propanol, isopropanol and butanol. Preferably, isopropanol and/or ethanol are used. Preferably, the washing solution comprises at least 5% alcohol and at least 0.1M chaotropic salt, preferably at least 0,5M, more preferred at least 0.8M chaotropic salt. However, also washing solutions without a chaotropic agent can be used. Furthermore, the washing solution may comprise a detergent. Preferably, ionic and/or non-ionic detergents are used as detergent. Preferably, a non-ionic detergent such as but not limited to Triton X100, Tween, Brij35 or NP-40 is used in a concentration of at least 0.1 %.

A further suitable washing solution which can be used alternatively or also in addition to the washing solutions described above comprises an alcohol and a buffer. Suitable alcohols and buffers such as biological buffers are described above. Preferably, isopropanol or ethanol, most preferred ethanol is used for this second washing step. Preferably, ethanol is used in a concentration of at least 70% v/v, preferably at least 80% v/v. The buffer is preferably Tris at a pH of approx. 7 to 8. According to one embodiment, the solution used for washing comprises at least one chaotropic agent, at least one alcohol, at least one detergent and/or at least one buffering component.

In case it is desired to perform an elution step to elute the nucleic acids from the solid phase, elution can be performed for example with classical elution solutions such as water, elution buffers, in particular biological buffers such as Tris and preferably elution solutions are used that do not interfere with the intended downstream application. After elution, the eluate can be heat denatured. However, it is also within the scope of the present invention to release and thus elute the nucleic acids from the solid phase by other elution means such as e.g. heating.

According to one embodiment, step c. comprises the following steps:

i. contacting the lysed sample or the nucleic acid containing portion obtained from the lysed sample with (aa) at least one chaotropic agent, preferably a chaotropic salt; (bb) at least one proteolytic enzyme, preferably proteinase K; and/or (cc) one or more salts, thereby providing a lysis mixture;

ii. binding nucleic acids comprised in the lysis mixture to a nucleic acid binding solid phase, wherein in step ii) optionally the binding conditions are adjusted by adding a binding composition;

iii. separating the solid phase with the bound nucleic acids from the remaining sample; and

iv. optionally washing the nucleic acids and

v. optionally eluting nucleic acids from the solid phase.

According to one embodiment, DNA as well as RNA is bound in step c. to a solid phase and thus can be isolated according to the method of the present invention. As discussed above, the teachings of the present invention increase the overall nucleic acid yield while preserving the integrity of the nucleic acids.

According to one embodiment, the sample comprises at least one non-target nucleic acid and at least one target nucleic acid and the method aims at isolating predominantly the target nucleic acid. E.g. the non-target nucleic acid can be DNA and the target nucleic acid can be RNA or vice versa.

According to one embodiment, isolation step c. comprises one or more intermediate steps in order to allow the isolation of predominantly the target nucleic acid. According to one embodiment, isolation step c. comprises not only an additional sample digestion step (see above) to additionally lyse the lysed sample that was digested in the presence of the cationic detergent or the nucleic acid containing portion obtained from the lysed sample but also an intermediate step that removes at least a portion of non-target nucleic acid. According to one embodiment, the non-target nucleic acid is destroyed by adding an appropriate enzyme which specifically destroys the non-target nucleic acid, e.g. a DNase if the target nucleic acid is RNA. Said enzyme can be added to the lysis or binding mixture or can be added after the nucleic acids were bound to a solid phase. Suitable embodiments for performing a respective non-target nucleic acid digestion step are known in the prior art and thus, do not need any further description here. According to one embodiment which is feasible if DNA and RNA is bound to the solid support, elution conditions selective for one type of nucleic acid, e.g. the RNA, can be applied to predominantly and thus selectively recover the target-nucleic acid from the solid support.

According to one embodiment, the non-target nucleic acid is removed by binding at least a portion of the non-target nucleic acid under appropriate conditions to a solid phase and then separating the non-target nucleic acid bound to the solid phase from the remaining sample comprising the target nucleic acid. This can be achieved e.g. by the addition of a suitable solid phase under conditions wherein mainly the non-target nucleic acids are bound to the solid phase. Suitable methods for selectively removing a non-target nucleic acid from a target nucleic acid are for example described in EP 0 880 537 and WO 95/21849. If desired, said non-target nucleic acid may also be further used, e.g. further processed such as e.g. eluted from the solid phase. However, it may also be discarded. When intending to isolate (only) RNA as target nucleic acid, the non-target nucleic acid is usually DNA.

In order to further reduce the amount of non-target nucleic acids in the isolated target nucleic acid, an intermediate step for degrading non-target nucleic acids using a suitable enzyme can be performed after at least the portion of the non-target nucleic acid was removed. It is also within the scope of the present invention to skip the removal step and to destroy non-target nucleic acids by using one or more appropriate enzymes only. Thus, according to one embodiment, isolation step c. comprises performing an enzymatic treatment in order to degrade non-target nucleic acids. According to one embodiment wherein RNA is isolated as target nucleic acid, a DNase treatment is performed. As the conditions for performing a DNase digest are well known in the prior art, they do not need further description here. Basically the same applies when isolating DNA as target nucleic acid and accordingly when using an RNase for degrading RNA as non-target nucleic acid.

According to one embodiment, the method according to the present invention is for isolating RNA, the sample is blood and the blood stabilization involved the use of at least one formaldehyde releaser and at least one anticoagulant and the method comprises the following steps:

a. obtaining the stabilized blood sample or a portion or fraction thereof wherein the portion or fraction of the stabilised blood sample preferably is selected from blood cells, serum or plasma;
b. contacting the stabilized blood sample or portion or fraction thereof with at least one cationic detergent and providing a lysed sample; and
c. isolating nucleic acids comprised in the lysed sample, wherein said isolated nucleic acids comprise or consist of RNA.

Preferably, in said embodiment

- the formaldehyde releaser is selected from a heterocyclic urea, diazolidinyl urea and/or imidazolidinyl urea, suitable and preferred concentrations are described above and
- in step b) the cationic detergent is selected from the group defined in claim 2 or a lysis composition as defined in claim 3 is used and wherein the composition comprising the stabilized sample or portion or fraction of the stabilized sample, the at least one cationic detergent and optionally one or more further lysis agents is incubated to provide the lysed sample; and

- step c) comprises the following steps:

i. contacting the lysed sample or a nucleic acid containing portion obtained from the lysed sample with one or more additional lysing agents thereby providing a lysis mixture, wherein preferably for this purpose the lysed sample or the nucleic acid containing portion obtained from the lysed sample is contacted with (aa) at least one chaotropic agent, preferably a chaotropic salt; (bb) at least one proteolytic enzyme; and/or (cc) one or more salts, thereby providing a lysis mixture;

- optionally removing DNA from the lysis mixture;

ii. adding alcohol to the lysis mixture to adjust the binding conditions and binding RNA to a nucleic acid binding solid phase; suitable and preferred binding conditions for isolating RNA in general and for isolating RNA comprising small RNA were described above and are preferably used in conjunction with said embodiment;
iii. separating the solid phase with the bound RNA from the remaining sample; and
iv. optionally washing the RNA and
v. optionally eluting RNA from the solid phase.

According to one embodiment wherein RNA is isolated from a sample comprising RNA and DNA, preferably from a stabilized blood sample or a portion or fraction thereof, isolation step c. comprises the following steps

i. contacting the lysed sample or the nucleic acid containing portion obtained from the lysed sample with (aa) at least one chaotropic agent, preferably a chaotropic salt; (bb) at least one proteolytic enzyme, preferably proteinase K; and/or (cc) one or more salts, thereby providing a lysis mixture;

ii. binding RNA to a solid phase, wherein at least one chaotropic agent and at least one alcohol in a concentration $\geq 30\%$ v/v is used during this binding step ii.,

iii. separating the solid phase with the bound RNA from the remaining sample; and

iv. optionally performing at least one washing step for washing the RNA bound to the solid phase, and

v. optionally eluting the RNA from the solid phase,

wherein said step c. optionally but preferably includes a step for removing at least a portion of the contained DNA using suitable means, preferably by removing at least a portion of the DNA from the lysis mixture prior to step ii. preferably by binding DNA to a solid phase using DNA selective binding conditions and separating the DNA bound to said solid phase from the remaining sample comprising the RNA which is then subjected to step ii. This embodiment is particularly suitable if the sample is a stabilized blood sample and RNA is the nucleic acid of interest. As described herein, it is also within the scope of the present invention to first obtain a portion or fraction of the stabilized blood sample, e.g. blood cells, serum or plasma and subjecting said portion or fraction of the stabilized blood sample to step b. Isolation step c. may also comprise additional steps, e.g. at least one additional enzymatic digestion step to digest DNA and/or protein contaminations.

According to a preferred embodiment of the present invention wherein at least RNA is isolated from a sample comprising at least RNA and DNA, preferably a stabilized blood sample, isolation step c. comprises the following steps:

- obtaining a lysed sample or a nucleic acid containing portion thereof, contacting it with a proteolytic enzyme and continuing the digestion preferably by incubating for at least 5min above room temperature preferably above 15°C, more preferred above 30°C, more preferred above 50°C. Suitable incubation conditions are described above, it is referred to the respective disclosure. This step, however, is optional if the lysed sample obtained from step b. is sufficiently digested e.g. due to the use of further lysis agents in step b..

- removing at least a portion of the DNA from the lysis mixture, preferably by binding DNA to a solid phase and separating the DNA bound to said solid phase from the remaining sample comprising the RNA. Thereby, the DNA can be removed. The removed DNA can be further processed, e.g. analysed or amplified. Optionally, the DNA is eluted from the solid phase if a parallel isolation of RNA and DNA is of interest.

- binding the RNA to a solid phase, wherein at least one chaotropic agent and at least one alcohol in a concentration ≥ 30% v/v is used during this RNA binding step. Suitable binding conditions and in particular suitable concentration ranges for the chaotropic agent and the alcohol are described above, it is referred to the respective disclosure. When intending to also isolate small RNA it is preferred to use an alcohol concentration ≥ 40% v/v, more preferred ≥ 50% v/v, most preferred ≥ 60% v/v.

- optionally performing at least one washing step for washing the RNA bound to said solid phase. Details with respect to the washing step were described above, it is referred to the respective disclosure.

- optionally performing a DNase digest and/or a digest using a proteolytic enzyme. Performing a DNase digest has the advantage that remaining traces of DNA can be efficiently removed. Performing a second protein digestion step is also advantageous in order to increase the purity of the isolated RNA. Preferably, the RNA is eluted prior to performing the DNase digest and the proteolytic enzyme is added after the DNase digest was performed and the reaction mixture is incubated in the presence of a chaotropic agent. Suitable digestion conditions are also described above. Preferably, proteinase K is used as proteolytic enzyme. Details with respect to said second protein digestion step and the associated advantages are described in EP 10 007 346.9. After the protein digestion step was performed, the RNA is re-bound to the second solid phase preferably by adding at least one chaotropic agent and at least one alcohol. Suitable binding conditions are described above, it is referred to the respective disclosure. Preferably, the same binding conditions are used that were used in the first RNA binding step. After rebinding, optionally one or more washing steps can be performed.

- optionally eluting RNA from said second phase and optionally denaturing the eluted RNA by performing a heat treatment.

It is also within the scope to perform additional intermediate steps than the ones described herein. However, according to certain embodiments, no additional steps other than the ones described herein are performed.

The term "sample" is used herein in a broad sense and is intended to include a variety of sources that contain nucleic acids. The sample may be a biological sample but the term also includes other, e.g. artificial samples which comprise nucleic acids. Exemplary samples include, but are not limited to, body fluids in general; whole blood; serum; plasma; red blood cells; white blood cells; buffy coat, tumor cells, fetal cells, host and graft cells; swabs, including but not limited to buccal swabs, throat swabs, vaginal swabs, urethral swabs, cervical swabs, throat swabs, rectal swabs, lesion swabs, abcess swabs, nasopharyngeal swabs, and the like; urine; sputum; saliva; semen; lymphatic fluid; liquor; amniotic fluid; cerebrospinal fluid; peritoneal effusions; pleural effusions; fluid from cysts; synovial fluid; vitreous humor; aqueous humor; bursa fluid; eye washes; eye aspirates; pulmonary lavage; lung aspirates; bone marrow aspirates, cells in suspension, tissues, including but not limited to, liver, spleen, kidney, lung, intestine, brain, heart, muscle, pancreas, cell cultures, as well as lysates, extracts, or materials obtained from any cells and microorganisms and viruses that may be present on or in a sample and the like. Materials obtained from clinical or forensic settings that contain nucleic acids are also within the intended meaning of the term sample. Furthermore, the skilled artisan will appreciate that lysates, extracts, or materials or portions thereof obtained from any of the above exemplary samples are also within the scope of the term sample. Preferably, the sample is a biological sample derived from a human, animal, plant, bacteria or fungi. In particular, the term "sample" refers to a nucleic acid containing sample which also comprises cells or is suspected of comprising cells. Preferably, the sample is selected from the group consisting of cells, tissue, bacteria, virus and body fluids such as for example blood, blood products such as buffy coat, plasma and serum, urine, liquor, sputum, stool, CSF and sperm, epithelial swabs, biopsies, bone marrow samples and tissue samples, preferably organ tissue samples such as lung and liver. According to one embodiment, the sample is a liquid sample. Preferably, the sample is selected from body fluids, samples comprising cells in suspension, bone marrow aspirates, urine, whole blood and blood products such as buffy coat, serum or plasma. Most preferred, the sample is selected from whole blood and blood products such as buffy coat, serum or plasma. When blood or a blood product is processed, the stabilization of the sample shall involve the use of an anticoagulant in addition to the formaldehyde releaser. Suitable embodiments are described above. According to one embodiment, the sample is not a tissue sample such as a solid tissue sample.

The term "nucleic acid" or "nucleic acids" as used herein, in particular refers to a polymer comprising ribonucleosides and/or deoxyribonucleosides that are covalently bonded, typically by phosphodiester linkages between subunits, but in some cases by phosphorothioates, methylphosphonates, and the like. Nucleic acids include, but are not limited to all types of DNA and/or RNA, e.g. gDNA; circular DNA; circulating DNA; hnRNA; mRNA; extracellular RNA, noncoding RNA (ncRNA), including but not limited to rRNA, tRNA, lncRNA (long non coding RNA), lincRNA (long intergenic non coding RNA), miRNA (micro RNA), siRNA (small interfering RNA), snoRNA (small nucleolar RNA), snRNA (small nuclear RNA) and stRNA (small temporal RNA), piRNA (piwi interacting RNA), tiRNA (transcription initiation RNA), PASR (promoter associated RNA), CUT (cryptic unstable transcripts); fragmented nucleic acid; nucleic acid obtained from subcellular organelles such as mitochondria or chloroplasts; and nucleic acid obtained from microorganisms, parasites, or DNA or RNA viruses that may be present in a biological sample. Small RNA or the term small RNA species in particular refers to RNA having a length of less than 500nt, 400nt, 300nt or 100nt and includes but is not limited to miRNA, siRNA, other short interfering nucleic acids, snoRNAs and the like. According to one embodiment, the nucleic acid to be isolated is RNA. The RNA may include small RNA species. As becomes apparent from the described examples of samples that can be processed according to the method of the present invention, a sample may comprise more than one type of nucleic acid. Depending on the intended use, it may be desirous to isolate all types of nucleic acids from a sample (e.g. DNA and RNA) or only certain types or a certain type of nucleic acid (e.g. only RNA but not DNA or *vice versa* or DNA and RNA are supposed to be obtained separately). Suitable methods for isolating either DNA or RNA or both types of nucleic acids in parallel are known in the prior art and are also described above.

Furthermore, the stabilized sample may be pretreated or processed prior to contacting the sample with the cationic detergent. The type of pretreatment or processing also depends on the type of sample that is processed. According to one embodiment, prior to lysis, nucleic-acid-containing cells are enriched from the stabilized sample, such as for example a stabilized blood sample. A variety of methods is available to the skilled person for this purpose, and these methods are in principle known per se in expert circles. Such suitable methods include for example the lysing or destroying of undesired cells in the mixture, the addition of surfaces onto which the cells of interest to be lysed adsorb or bind, filtration, sedimentation, or centrifugation steps or a combination of a plurality of these methods, without being limited thereto. The preferred object is to separate the cells which contain the genetic material to be analysed from other cells or contaminating additives, or to enrich them with respect to the other additives.

According to one embodiment, blood samples are first treated with an erythrocyte lysis buffer so that the erythrocytes in the sample are lysed. Then, the remaining white blood cells are separated from the lysis mixture e.g. assisted by centrifugation. The supernatant is discarded and the white blood cells are immediately available for further processing using the lysis method according to the present invention. For lysing erythrocytes a lysis buffer is used which serves exclusively for lysing erythrocytes which are present in blood. Any erythrocyte lysis buffer can be used for this purpose. One suitable example is the lysis buffer ELB1 (320 mM sucrose, 50 mM Tris/Cl pH 7.5, 5 mM $MgCl_2$, 1% Triton X-100) or ELB2 (155 mM $NH_4Cl$, 10 mM $KHCO_3$).

According to one embodiment, the sample is brought into contact with a surface onto which the cells of interest from which the nucleic acids are to be isolated adsorb or bind. Thereafter, the remaining constituents of the sample are largely removed by a separation method. To this end, the sample is brought into contact with a suitable surface onto which the cells adsorb or bind. Examples of such surfaces which are suitable for this purpose are small spheres, also known as beads, for example made of glass, silica, polymers or coated beads, preferably magnetic beads. However, it is also possible to modify the surfaces of the consumables such as, for example, reaction vessels, reaction filters, filter columns, spin filter minicolumns, membranes, frits, glass fibre fabric, dishes, tubes, (pipette) tips or wells of multiwell plates for the binding. Functional groups that are suitable to promote binding of cells are well-known and include anionic and/or cationic exchange moieties. Furthermore, if the binding and/or isolation of specific cells is intended, the surface can also be functionalised with ligands such as e.g. antibodies which specifically bind a certain cell type. In an especially preferred embodiment, magnetic beads are used. For the lysis of erythrocytes, a surface can be introduced in the form of modified magnetic particles. The white blood cells adsorb onto the surfaces and can be enriched by magnetic separation. Beads which are suitable for said purposes may comprise any type of magnetic beads known to date in which a magnetic core is coated with a glass or polymer coating and which on their surface bear groups which make possible an unspecific attachment or binding of nucleic-acid-containing cells onto the beads. Suitable functional groups which promote binding are known in the prior art. It is preferred to employ those beads which are hydrophilic on their surface, for example which bear acid groups, preferably carboxylic acid groups, phosphoric acid groups or sulfuric acid groups, or their salts, more preferably carboxylic acid groups or their salts. For the abovementioned groups it is possible to be bound directly to the surface or to be part of the polymer which forms the surface coating, to be bound to the surface via spacer molecules or to be parts of a compound which is bound to the surface of the beads. In one embodiment, the beads bear on their surface a total charge which is weakly negative in overall terms, since the cells are bound particularly effectively when such conditions prevail. A neutral to weakly positive charge of the beads is also possible. Examples of suitable carboxylated polymers which are suitable as coating material for the beads and which provide a surface which is suitable for the invention are described in detail in the German patent application DE 10 2005 040 259.3. Examples of compounds which may be bound to the surface are glycine, hydrazine, aspartic acid, 6-aminocaproic acid, NTA (nitrilotriacetic acid), polyacrylic acid (PAA), glycerin, diglyme (diethylene glycol dimethyl ether), glyme (dimethoxyethane), pentaerythritol, toluene or combinations of these, without being limited thereto. Likewise suitable magnetic beads are those which are described in the German patent application DE 10 2005 058 979.9. Such suitable magnetic beads are commercially available. Further examples of suitable beads are silica beads, such as, for example, MagAttract Suspension-B, MagAttract Suspension-G (all by Qiagen). The cells are brought into contact with the magnetic beads over a sufficiently long period of time, i.e. a period of time which suffices to allow the cells to bind/attach themselves to the beads. Such a period of time should be at least 30 s, preferably at least 1 min, further preferably at least 3 min. If magnetic particles are employed in the lysis of the erythrocytes, a magnetic separation may be performed prior to removing the lysate for the subsequent detection reaction, in order to avoid carry-over of magnetic particles. However, beads may remain in the mixture, but should preferably not be carried over into the analysis method. DNA and RNA are present in the lysate in free form because the lysate does not have any properties which support the binding of nucleic acid to the beads.

After the attachment/binding of the cells, e.g. white blood cells, to the magnetic particles, the cells can be collected or separated from the medium which surrounds the cells by applying a magnetic field to the vessel in which the cells together with the beads are located. In a preferred embodiment, a magnet is applied externally to the vessel in which the cells and the magnetic beads are located, and the remainder of the sample is removed from the vessel, for example decanted off, or removed with the aid of a suitable device, for example drawn off with the aid of a pipette. The magnetic particles together with the cells can optionally be resuspended in a suitable wash medium and thereby washed, where after a magnetic field is again applied to the vessel and the wash medium is again removed from the vessel. The present method therefore provides a particularly gentle handling of the cells.

After the enrichment of the nucleic-acid-containing cells to be lysed, the collected cells are lysed using the method according to the present invention as described in detail hereinabove. For lysis, a cationic detergent is added. Furthermore, also the details of the nucleic acid isolation are described in detail above.

According to one embodiment, the method comprises the following steps:

 a. obtaining white blood cells from a stabilized blood sample;

 b. contacting the white blood cells with at least one cationic detergent for lysis and providing a lysed sample; and

 c. isolating cellular nucleic acids.

Red blood cells may be lysed prior to step a.. Preferably, the nucleic acid to be isolated is RNA and the stabilization of the blood sample involved the use of a formaldehyde releaser and an anticoagulant. Suitable embodiments are described above it is referred to the above disclosure which also applies here.

Details with respect to the individual steps a. to c. were also described above. It is referred to the respective disclosure.

The present invention also pertains to the use of a cationic detergent during lysis of a stabilized sample or portion or fraction thereof in preparation for nucleic acid isolation, wherein the sample stabilization involved the use of at least one formaldehyde releaser. Details with respect to the cationic detergent, potential lysis compositions comprising a cationic detergent, stabilization compositions and procedures as well as suitable and preferred samples and nucleic acid isolation procedures were described in detail above. It is referred to the respective disclosure.

Preferably, said use is characterized by one or more of the following features:

 a) the cationic detergent has one or more of the characteristics as defined in claim 2;
 b) a lysis composition according to claim 3 is used; and/or
 c) the sample has been stabilized as defined in one or more of the claims 9 to 12.

Preferably, in conjunction with the use according to the present invention, the sample or portion or fraction thereof has one or more of the following characteristics:

 a) it comprises cells;
 b) it is selected from the group consisting of whole blood, plasma, serum, lymphatic fluid, urine, liquor, ascites, milk, stool, bronchial lavage, saliva, bone marrow aspirates, amniotic fluid, semen/seminal fluid, swabs/smears, body fluids, body secretions, nasal secretions, vaginal secretions, wound secretions and excretions, bone marrow aspirates, cell suspensions, cell culture and cell culture supernatants;
 c) it is a cell-free, cell-depleted or cell containing body fluid sample or portion or fraction thereof; and/or
 d) it is whole blood.

As discussed above, the nucleic acid isolation methods described herein are particularly useful for isolating RNA from blood samples or portions or fractions thereof that were stabilized by using at least one formaldehyde releaser and an anticoagulant.

The isolated nucleic acids may after isolation be further processed and/or analysed. For example they can be modified, contacted with at least one enzyme, amplified, reverse transcribed, cloned, sequenced, contacted with a probe and/or be detected. In particular the isolated nucleic acid such as for example the cellular RNA and/or the extracellular nucleic acids can be tested to identify the presence, absence or severity of a disease state. Therefore, the methods according to the present invention further contemplate a step of nucleic acid testing. Here, basically any standard testing method can be used. The analysis/further processing of the nucleic acids can be performed, e.g., using any nucleic acid analysis/processing method including, but not limited to amplification technologies, polymerase chain reaction (PCR), isothermal amplification, reverse transcription polymerase chain reaction (RT-PCR), quantitative real time polymerase chain reaction (Q-PCR), digital PCR, gel electrophoresis, capillary electrophoresis, mass spectrometry, fluorescence detection, ultraviolet spectrometry, hybridization assays, DNA or RNA sequencing, restriction analysis, reverse transcription, NASBA, allele specific polymerase chain reaction, polymerase cycling assembly (PCA), asymmetric polymerase chain reaction, linear after the exponential polymerase chain reaction (LATE-PCR), helicase-dependent amplification (HDA), hot-start polymerase chain reaction, intersequence-specific polymerase chain reaction (ISSR), inverse polymerase chain reaction, ligation mediated polymerase chain reaction, methylation specific polymerase chain reaction (MSP), multiplex polymerase chain reaction, nested polymerase chain reaction, solid phase polymerase chain reaction, or any combination thereof. Respective technologies are well-known to the skilled person and thus, do not need further description here. According to one embodiment, the isolated nucleic acids are analysed, e.g. to identify, detect, screen for, monitor or exclude a disease or a predisposition for a disease, an infection and/or at least one fetal characteristic. Furthermore, the isolated nucleic acids can be analysed e.g. for profiling the isolated nucleic acids, for determining nucleic acid biomarkers, for diagnostic purposes in general, in particular molecular diagnostic purposes.

Furthermore, as described above, it is also within the scope to isolate cells from the stabilized sample and to analyse the cells. Respective cell analysis methods are known in the prior art and are also described above. A respective cell analysis can be performed in addition to isolating nucleic acids from said cells.

Numeric ranges are inclusive of the numbers defining the range. The headings provided herein are not limitations of the various aspects or embodiments of this invention which can be read by reference to the specification as a whole. The term "solution" as used herein, in particular refers to a liquid composition, preferably an aqueous composition. It may be a homogenous mixture of only one phase but it is also within the scope of the present invention that a solution that is used according to the present invention comprises solid components such as e.g. precipitates. According to one embodiment, subject matter described herein as comprising certain steps in the case of methods or as comprising certain ingredients in the case of compositions, solutions and/or buffers refers to subject matter consisting of the respective steps or ingredients. It is preferred to select and combine preferred embodiments described herein and the specific subject-matter arising from a respective combination of preferred embodiments also belongs to the present disclosure.

## BRIEF DESCRIPTION OF THE FIGURES

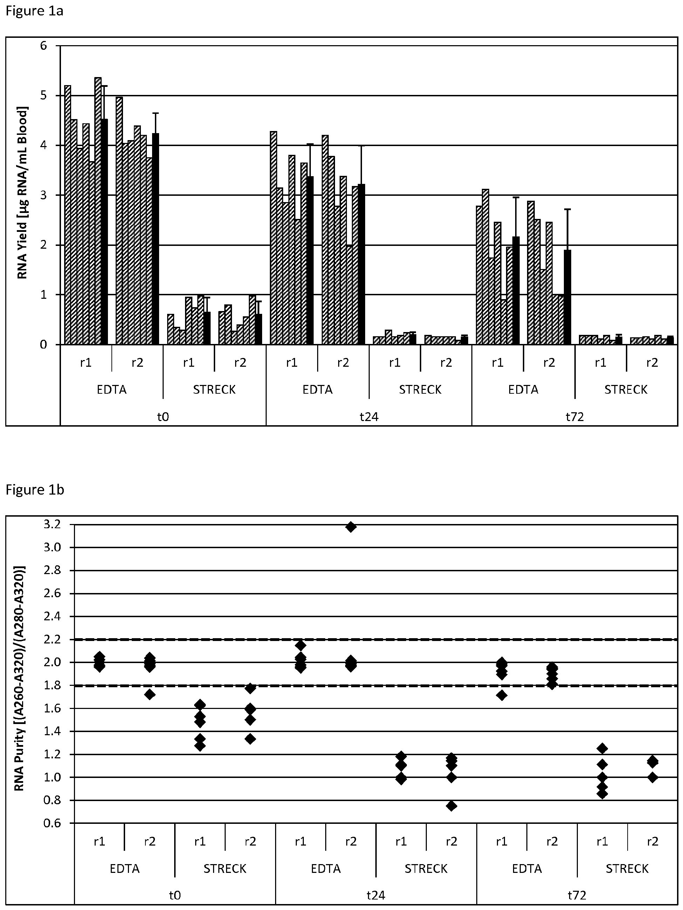

Figure 1 is a graphic representation showing (a) yield and (b) purity of RNA samples from peripheral blood of six donors directly drawn into BD EDTA tubes (EDTA) and STRECK Cell-Free RNA BCT tubes (STRECK). Blood samples were processed from duplicate tubes (r1: first replicate tube, r2: second replicate tube) without incubation (t0) and after incubation at room temperature for one (t24) and three days (t72) as described in example 1.

(a) RNA yields of individual samples are shown as striped bars and means as black solid bars with standard deviations.
(b) RNA purities of individual samples are shown as black solid diamonds. The typical range of pure RNA (1.8-2.2) is indicated with dashed lines.

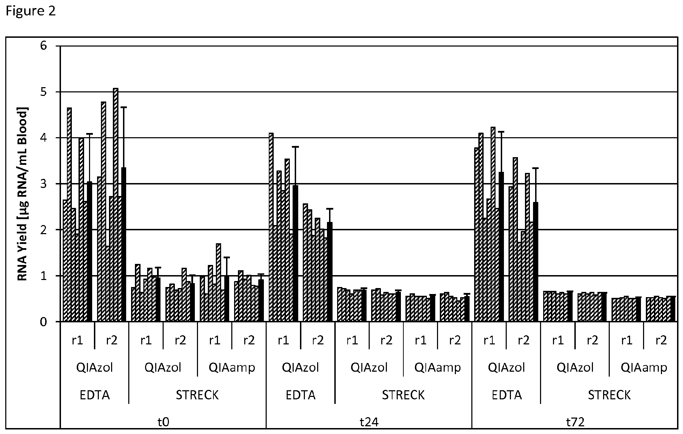

Figure 2 is a graphic representation showing yield of RNA samples from peripheral blood of six donors directly drawn into BD EDTA tubes (EDTA) and STRECK Cell-Free RNA BCT tubes (STRECK). Blood samples were processed from duplicate tubes (r1: first replicate tube, r2: second replicate tube) without incubation (t0) and after incubation at room temperature for one (t24) and three days (t72). RNA from blood samples was prepared with two protocols as described in example 2: Phenol based extraction with silica membrane based clean-up protocol (QIAzol protocol) and silica membrane based extraction protocol using the QIAGEN QIAamp RNA Blood Mini Kit (QIAamp protocol). RNA yields of individual samples are shown as striped bars and means as black solid bars with standard deviations.

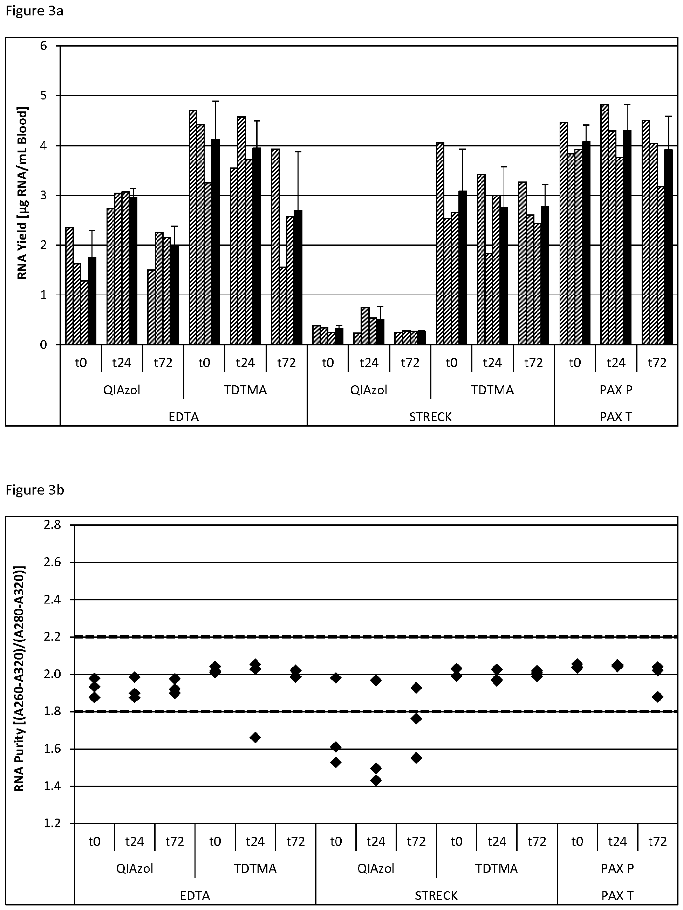

Figure 3 is a graphic representation showing (a) yield and (b) purity of RNA samples from peripheral blood of three donors directly drawn into BD EDTA tubes (EDTA), STRECK Cell-Free RNA BCT tubes (STRECK) and PreAnalytiX PAXgene Blood RNA Tubes (PAX T). Blood samples were processed from replicate tubes without incubation (t0) and after incubation at room temperature for one (t24) and three days (t72). RNA from blood samples was prepared with three protocols as described in example 3: Phenol based extraction with silica membrane based clean-up protocol (QIAzol protocol), silica membrane based protocol using the PreAnalytiX PAXgene Blood RNA Kit (PAX P) and a protocol in accordance with the present invention (TDTMA protocol).

(a) RNA yields of individual samples are shown as striped bars and means as black solid bars with standard deviations.

(b) RNA purities of individual samples are shown as black solid diamonds. The typical range of pure RNA (1.8-2.2) is indicated with dashed lines.

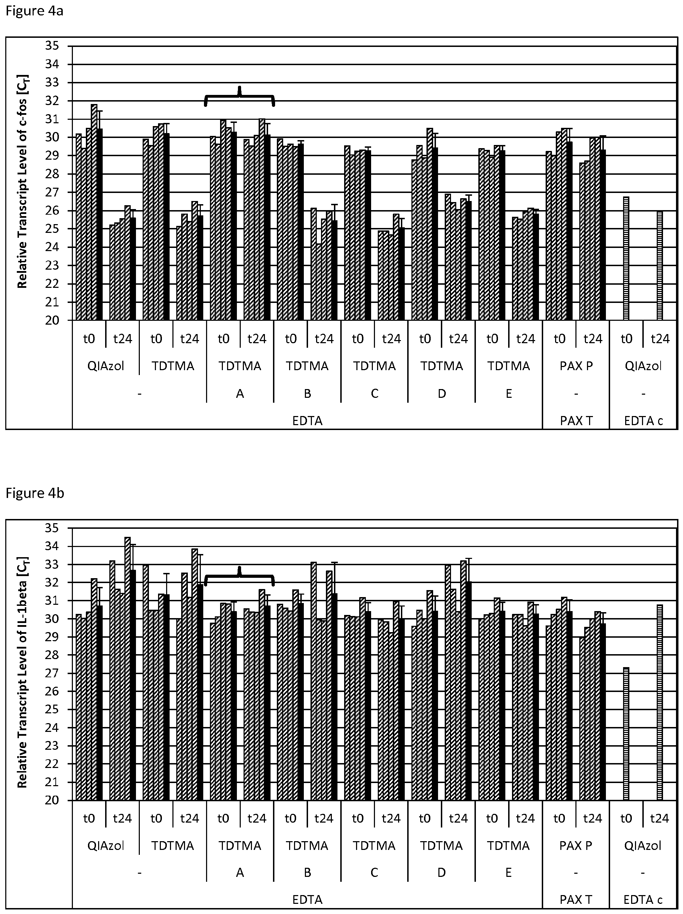

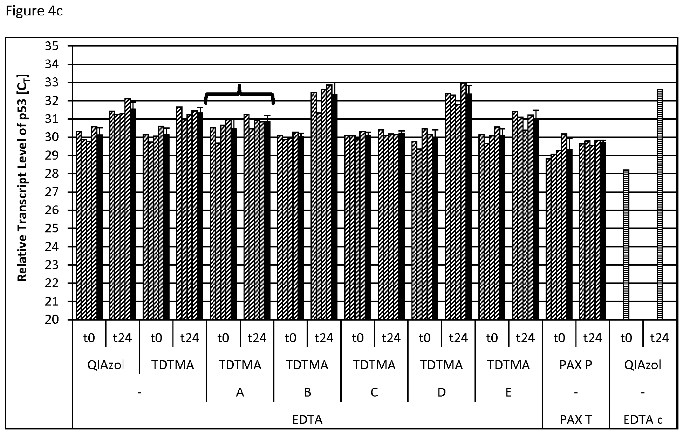

Figure 4 is a graphic representation showing relative gene transcript levels of (a) c-fos, (b) IL-1beta and (c) p53 of RNA samples from peripheral blood of four donors. Blood was directly drawn into BD EDTA tubes (EDTA, EDTA c) and PreAnalytiX PAXgene Blood RNA Tubes (PAX T). Blood samples were kept untreated (-) or were treated with different test solutions (A, B, C, D, E) as described in example 4. Blood samples were processed from replicate tubes without incubation (t0) and after incubation at room temperature for one day (t24). RNA was prepared with three protocols as described in example 4: Phenol based extraction with silica membrane based clean-up protocol (QIAzol protocol), silica membrane based protocol using the PreAnalytiX PAXgene Blood RNA Kit (PAX P) and a protocol in accordance with the present invention (TDTMA protocol). Relative transcript levels given as cycle thresholds ($C_T$) of individual samples are shown as striped bars, control samples from an additional donor of a different experiment (EDTA c) as horizontally striped bars and means as black solid bars with standard deviations. Lower $C_T$ values indicate gains, whereas higher values indicate losses of transcripts over time of blood sample storage. Results of transcript level stabilization consistently achieved with test solution A for all three transcripts analyzed are highlighted with horizontal brackets.

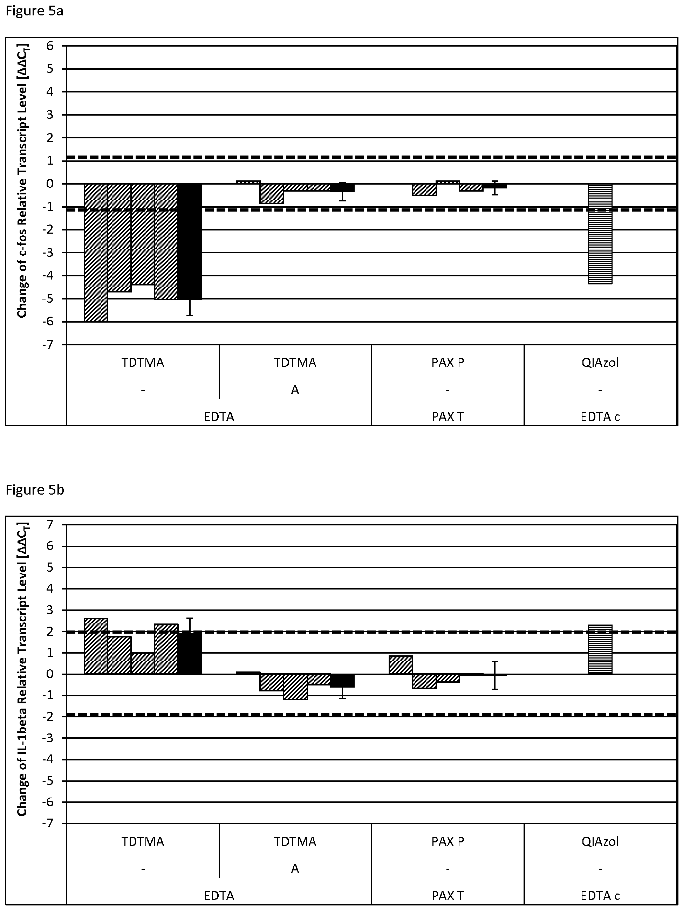

Figure 5 is a graphic representation showing changes of the relative gene transcript levels of (a) c-fos and (b) IL-1beta as the result of blood sample storage for one day at room temperature (comparison of t0 and t24 samples). A subset of RNA samples of the experiment shown in figure 4 was subjected to duplex RT-PCR analysis utilizing 18S rRNA in parallel to c-fos and IL-1 beta to calculate relative changes of transcript levels as follows:

$$\Delta\Delta\, C_T \;=\; \Delta\, C_T(t0) \;-\; \Delta\, C_T(t24),$$

with

$$\Delta\, C_T(t0) \;=\; C_T(18S\ rRNA,\ t0) \;-\; C_T(\text{c-fos or IL-1beta},\ t0)$$

$$\Delta\, C_T(t24) \;=\; C_T(18S\ rRNA,\ t24) \;-\; C_T(\text{c-fos or IL-1beta},\ t24)$$

Peripheral blood of four donors was directly drawn into BD EDTA tubes (EDTA, EDTA c) and PreAnalytiX PAXgene Blood RNA Tubes (PAX T). Blood samples were kept untreated (-) or were treated with test solution A as described in example 4 and shown in figure 4. Blood samples were processed from replicate tubes without incubation (t0) and after incubation at room temperature for one day (t24). RNA was prepared with three protocols as described in example 4: Phenol based extraction with silica membrane based clean-up protocol (QIAzol protocol), silica membrane based protocol using the PreAnalytiX PAXgene Blood RNA Kit (PAX P) and a protocol in accordance with the present invention (TDTMA protocol).

Transcript level differences given as $\Delta\Delta\, C_T$ of individual sample pairs (t0, t24) are shown as striped bars, control sample pair from an additional donor of a different experiment (EDTA c) as a horizontally striped bar and means as black solid bars with standard deviations.

Negative $\Delta\Delta$ CT values indicate gains, whereas positive values indicate losses of transcripts over time of blood sample storage. Significant transcript level changes are identified by $\Delta\Delta\, C_T$ outside the assays precision as established with method validation experiments and calculated with 3x sigma. The assays precision are indicated by black dashed lines (-1.16 > $\Delta\Delta\, C_T$ > 1.16 for c-fos and -1.98 > $\Delta\Delta\, C_T$ > 1.98 for IL-1 beta).

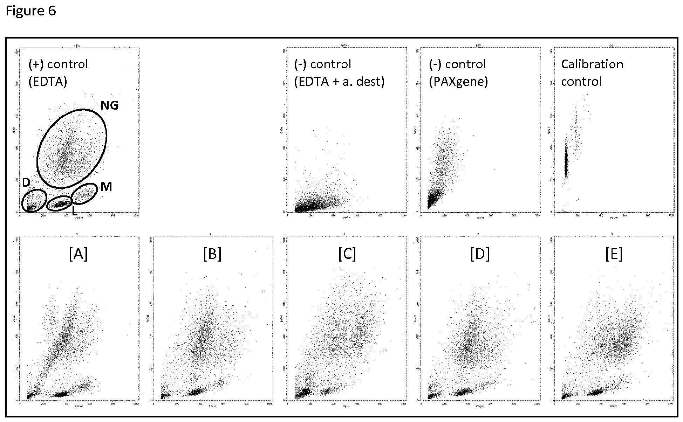

Figure 6 is a graphic representation showing the results of flow cytometry (FC) analysis of peripheral blood samples of one donor of the experiment shown in figure 4 and 5.

Blood was directly drawn into BD EDTA tubes and PreAnalytiX PAXgene Blood RNA Tubes (PAXgene). Replicate EDTA blood samples were kept untreated ([+] control [EDTA], [-] control [EDTA + a. dest]) or were treated with five different test solutions (A, B, C, D, E) as described in example 4. All blood samples received an incubation of one day at room temperature prior to FC analysis.

Shown per picture are the size (x-axis = forward scatter) and granularity (y-axis = sideward scatter) of 10,000 events per sample tested that contain signals of cells, cell debris and particles. The different populations of white blood cells that are distinguishable from each other in FC analysis (L, M, NG) and the cell-free fraction (D) are indicated by circles for the (+) control (EDTA) sample. They are as follows:

D = Debris, subcellular components, fragments of cells
L = Lymphocytes
M = Monocytes
NG = Neutrophilic granulocytes
(+) control (EDTA): Untreated EDTA blood sample serving as a positive control of cell stabilization.
(-) control (EDTA + a. dest): Untreated EDTA blood sample that was completely lysed by addition of deionized water serving as a negative control of cell stabilization.
(-) control (PAXgene): PAXgene blood sample that contains completely lysed cells as all cells get directly lysed during blood collection into the tube as soon as blood gets into contact with the RNA stabilization additititve in the tube. This sample served as an additional negative control of cell stabilization.
Calibration control: CALIBRITE Beads (BD) of known fluorescence and diameter serving to calibrate the BD FACSCalibur™ instrument.
[A], [B], [C], [D], [E]: EDTA blood samples treated with test solutions A, B, C, D, E

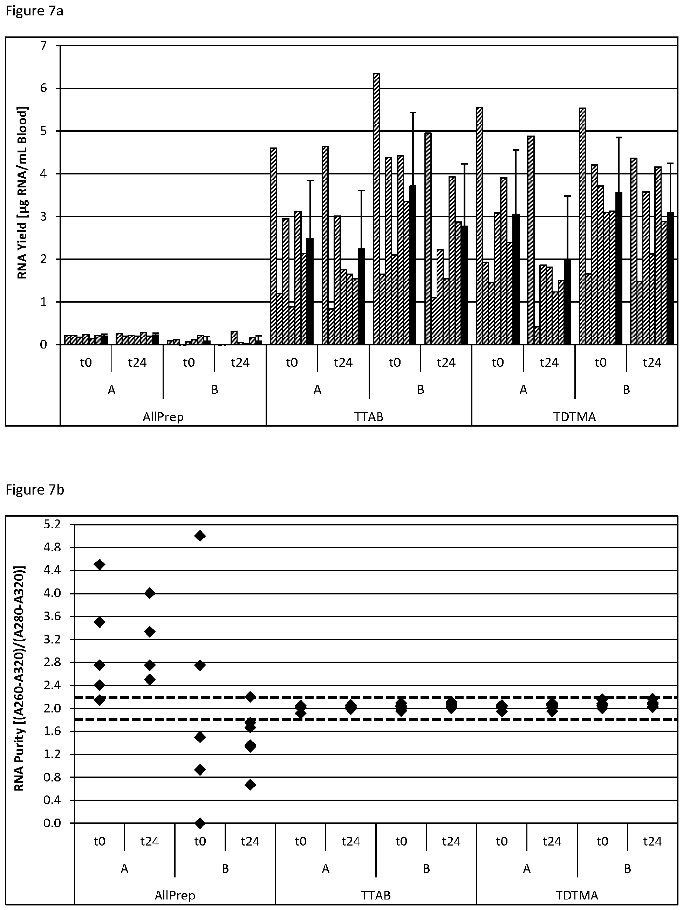

Figure 7 is a graphic representation showing (a) yield and (b) purity of RNA samples from peripheral blood of six donors directly drawn into BD EDTA tubes. Replicate blood samples were treated with two test solutions (A, B) and processed without incubation (t0) and after incubation at room temperature for one day (t24). RNA from blood samples was prepared with three protocols as described in example 5: Erythrocyte lysis, followed by silica membrane based isolation protocol using the QIAGEN AllPrep DNA/RNA Mini Kit (AllPrep protocol) and two protocols in accordance with the present invention (TTAB protocol, TDTMA protocol).

(a) RNA yields of individual samples are shown as striped bars and means as black solid bars with standard deviations.
(b) RNA purities of individual samples are shown as black solid diamonds. The typical range of pure RNA (1.8-2.2) is indicated with dashed lines.

## EXAMPLES

### Example 1

Peripheral blood samples from six donors were directly drawn into BD EDTA tubes (EDTA) and STRECK Cell-Free RNA BCT tubes (STRECK). Blood samples were processed both without storage (t0) and after incubation for one day (t24) and three days (t72) at room temperature from duplicate tubes (r1: first replicate tube, r2: second replicate tube) at each test time point. RNA isolation and purification was done using a phenol based extraction combined with a silica membrane based clean-up protocol. In detail, 1ml of blood was thoroughly mixed with 1ml QIAzol (QIAGEN). After addition of 200$\mu$l chloroform and additional mixing, samples were centrifuged at 4°C to separate aqueous from organic solvent phase. 1ml of the RNA containing aqueous phase was mixed with 0.5ml of ethanol (absolute) and the mixture was applied to RNeasy Mini Kit spin columns (QIAGEN). RNA was bound to the silica membrane by centrifugation and further purification was performed with application of wash buffers of the RNeasy Mini Kit according to the handbook (4th edition, September 2010) including a DNase treatment step to remove traces of genomic DNA. RNA was finally eluted from the spin column membrane by using two fractions of 40$\mu$l each of buffer BR5 of the PAXgene Blood RNA Kit (PreAnalytiX).

The yield and purity of the isolated RNA was determined from RNA aliquots using the SpectraMax plus UV spectrophotometer (Molecular Devices) that was properly zeroed using the same dilution of elution buffer that was used to dilute the RNA. RNA was diluted with 10 mMTris-HCl pH 7.6. Background absorption at 320 nm was subtracted from the absorption at 260 and 280nm. RNA purity was calculated as absorbance ratio (A260 - A320) / (A280 - A320) and RNA yield as (A260 - A320) $\times$ 44$\mu$g/ml $\times$ dilution factor $\times$ elution volume ($\mu$l) / extracted blood volume (ml).

The results of this example are shown in figure 1. Blood samples in EDTA tubes generated much higher RNA yields than those in STRECK tubes using the identical RNA preparation protocol: 7.0, 18.9 and 13.9 fold difference on average in t0, t24 and t72 samples, respectively. Moreover RNA yields dropped over time of blood sample storage, resulting in RNA yields of below 200ng RNA /1 ml blood in STRECK t72 samples. Rounded values of RNA purity of EDTA samples was in the typical range of highly pure RNA (1.8-2.2) for all samples, except one, whereas STRECK samples demonstrated a much lower number of highly pure RNA: EDTA 35/36 (97.2%) vs. STRECK 1/36 (2.8%).

The results demonstrate that from blood in EDTA tubes, but not from blood in formaldehyde releasing agent containing STRECK tubes, typical amounts of RNA can be prepared using the protocol as described. RNA prepared from STRECK tubes with the protocol used are expected not to be of sufficient yield and purity to be used in several RNA based downstream assays. Moreover, due to the low absorption values of the STRECK samples, the measurements of the RNA purity were not reliable.

**Example 2**

The experiment described in example 1 indicated that isolation and purification of RNA from blood samples treated with RNA preservation and stabilization solution known to contain the formaldehyde releaser DU (STRECK samples) with a phenol based protocol did not result in sufficient amounts and purity of RNA. Therefore, a second procedure of RNA preparation from blood was tested which is widely used as a standard RNA preparation procedure from blood whether it results in RNA suitable for analysis.

Peripheral blood samples from six donors were directly drawn into BD EDTA tubes (EDTA) and STRECK Cell-Free RNA BCT tubes (STRECK). Blood samples were processed both without storage (t0) and after incubation for one day (t24) and three days (t72) at room temperature from duplicate tubes (r1: first replicate tube, r2: second replicate tube) each test time point. RNA isolation and purification was done using a phenol based extraction combined with a silica membrane based clean-up protocol (QIAzol protocol) as described in example 1 and with a silica membrane based extraction protocol using the QIAGEN QIAamp RNA Blood Mini kit according to the handbook (2nd edition, April 2010) including the optional on-column DNase digestion step (QIAamp protocol).

The yield and purity of the prepared RNA was determined from RNA aliquots as described in example 1. The results of this example are shown in figure 2. As shown before in example 1, in this example blood samples in EDTA tubes generated much higher RNA yields than those in STRECK tubes using the identical RNA preparation protocols. Moreover RNA yields dropped over time of blood sample storage. RNA yields from STRECK tubes were comparable low with both, QIAzol and QIAamp protocols.

The results demonstrate that from blood in EDTA tubes, but not from blood in formaldehyde releasing agent containing STRECK tubes, typical amounts of RNA can be prepared using the QIAzol protocol as described. Both, the QIAzol and the QIAamp protocol are inefficient to prepare RNA from blood collected into STRECK tubes.

**Example 3**

As both protocols utilized in experiments described in example 1 and example 2 failed to generate sufficient quantities and qualities of RNA from blood samples collected into STRECK tubes, optimized new RNA protocol was developed and tested. Peripheral blood samples from three donors were directly drawn into BD EDTA tubes (EDTA), STRECK Cell-Free RNA BCT tubes (STRECK) and PreAnalytiX PAXgene Blood RNA Tubes (PAX T). Blood samples were processed both without storage (t0) and after incubation for one day (t24) and three days (t72) at room temperature from replicate tubes. RNA isolation and purification was done using three different protocols:

(1) QIAzol: The QIAzol protocol described in example 1 was applied to blood samples collected into EDTA and STRECK tubes.
(2) PAX P: Blood samples directly drawn into PreAnalytiX PAXgene Blood RNA Tubes were processed with the PAXgene Blood RNA Kit according to the instructions of the handbook (Version 2, April 2008). These samples served as control samples, because said stabilisation tubes are specifically designed to ensure the preservation of RNA and allow the preparartion of RNA from the respectively stabilized sample with excellent results with respect to RNA quantity and quality.
(3) TDTMA - Method according to the present invention: The TDTMA protocol was applied to blood samples collected into EDTA and STRECK tubes. Aliquots of 2.5 ml blood each were contacted after the indicated incubation times (t0, t24, t72) with 6.9 ml of a lysis composition comprising a cationic detergent (here: tetradecyltrimethylammonium oxalate and a proton donor at pH 3.7). The mixture was incubated at room temperature for 6 hours. The lysed samples were stored at -20°C upon batchwise processing. After thawing, mixtures were centrifuged to harvest complexes of nucleic acids and detergents. Resulting pellets were processed further with the PreAnalytiX PAXgene Blood RNA Kit according to the kit handbook (Version 2, April 2008).

The yield and purity of the prepared RNA was determined from RNA aliquots as described in example 1. The results of this example are shown in figure 3. Blood samples in EDTA tubes generated much higher RNA yields than those in STRECK tubes using the QIAzol protocol for RNA preparation. These data confirmed the results obtained in previous experiments that are shown in example 1 and example 2 and served as experimental control to demonstrate insufficient yields with this protocol. Blood samples in STRECK tubes processed with the TDTMA protocol generated much higher and sufficient yields than replicate samples processed with the QIAzol protocol: 9.5, 5.4 and 10.4 fold difference on average in t0, t24 and t72 samples, respectively. Use of the TDTMA protocol with EDTA blood samples resulted also in sufficient amounts of RNA as well as PAX T samples processed with the PAX P protocol that served as a control of RNA stabilization and an additional control of a successful RNA preparation protocol.

RNA purity was in the typical range of highly pure RNA (1.8-2.2) for all samples, except one EDTA sample processed with the TDTMA protocol and the majority of STRECK samples processed with the QIAzol protocol: 5/9 (55.5% of rounded values) of all STRECK-QIAzol samples resulted in impure RNA.

The results demonstrate that the protocol according to the present invention allows to isolate RNA from DU treated blood samples by contacting the stabilized blood with a lysis composition comprising a cationic detergent and subsequent RNA preparation e.g. with the PAXgene Blood RNA Kit. Use of this protocol (TDTMA protocol) results in typical amounts of pure RNA, while the QIAzol protocol was again inefficient to prepare pure RNA from DU stabilized blood (blood collected in STRECK tubes).

## Example 4

A test system was established that allows the identification of solutions and/or reagent compositions that have gene transcript stabilization capabilities, as indicated by constant levels of transcripts from selected genes (c-fos, IL-1beta, p53). These transcripts were identified in prior studies as very unstable transcripts, which are induced or down regulated (gains and losses of transcripts) within minutes after blood collection and were therefore chosen as "worst case" markers for screening purposes.

Moreover, the experimental setup included flow cytometry (FC) as a method that indicates possible preservation of cells by the tested stabilization reagents by means of visualization of all different subpopulations of white blood cells (WBC).

To screen several reagents for potential stabilization properties, the following study setup was chosen: Replicate samples of peripheral whole blood from four donors were directly drawn into BD EDTA tubes to immediately prevent blood coagulation and into PreAnalytiX PAXgene Blood RNA Tubes (PAX T) resulting in direct cell lysis serving as control samples of proven RNA stabilization ([+] control of RNA stabilization) and complete cell lysis ([-] control of cell stabilization). To test potential cell and RNA stabilization solutions, aliquots of EDTA blood were mixed with test solutions (A-E) within short time (maximum of 15 minutes) post collection or were kept untreated (-). The latter samples served as control samples of known ex vivo cell integrity ([+] control of cell stabilization), but transcript level changes ([-] control of RNA stabilization) as the result of blood sample storage.

Replicate EDTA blood aliquots were directly contacted with the TDTMA solution of the TDTMA protocol (t0 samples) or after incubation for one day at room temperature (t24 samples), followed by RNA preparation with the TDTMA protocol as described below. Untreated EDTA blood aliquots were additionally processed directly or after incubation as mentioned with the QIAzol protocol as described below. Blood sample replicates in PAXgene Blood RNA Tubes (PAX T) were processed directly or after incubation as mentioned with the PAX P protocol as described below. One additional pair of EDTA blood samples from a separate experiment with a different donor was kept untreated and was processed with the QIAzol protocol immediately and after 24 hours of storage. It served as an additional control of unstabilized RNA during sample storage at room temperature (EDTA c).

Different chemicals were selected for a screening approach to test for possible transcript and cell stabilization properties. One volume of the following test solutions were mixed with 50 volumes of whole blood (200μl test solution added to 10ml of blood):

(A) 25% w/v diazolidinyl urea (DU)
(B) 1.5% w/v aurintricarboxylic acid
(C) 0.8% w/v sodium fluoride
(D) 10% w/v EDTA
(E) 4.5% w/v glyceraldehyde.

RNA isolation and purification was performed using three different protocols:

(1) QIAzol: The QIAzol protocol described in example 1 was applied to untreated (-) blood samples collected into EDTA tubes (EDTA, EDTA c)
(2) TDTMA - Method according to the present invention: The TDTMA protocol described in example 3 was applied to blood samples collected into EDTA tubes (EDTA) that were kept untreated (-) or treated with test solutions (A, B, C, D, E).
(3) PAX P: The PAX P protocol described in example 3 was applied to blood samples collected into PAXgene Blood RNA Tubes (PAX T) that were kept untreated (-).

All RNA samples were subjected to realtime RT-PCR using monoplex assays of c-fos, IL-1beta, p53 (see Figure 4) and duplex assays of c-fos/18S rRNA and IL-1beta/18S rRNA (see Figure 5). Monoplex assays were performed using the same amount of total RNA for all samples in order to compare relative transcript levels. Resulting $C_T$ values reflecting the relative amount of transcripts were directly compared between time points (see Figures 4a to 4c) or in case of the duplex assays were calculated as $\Delta C_T$ ($\Delta C_T$[t0], $\Delta C_T$[t24]) that served to calculate $\Delta\Delta C_T$ ($\Delta C_T$[t0] - $\Delta C_T$[t24]) indicating changes of relative transcript levels as the result of blood sample storage (see legend of figure 5 for details).

The results of this example are shown in figure 4 and figure 5. Blood samples of one donor as described above were subjected to flow cytometry analysis (FC) after one day of storage to analyze the integrity of white blood cells (WBC). Untreated EDTA blood was considered as positive control sample, as different populations of WBC remained visible in the EDTA tubes for this storage time. The aim of the analysis was to investigate the integrity of cells after treatment with different test solution (A-E).

As shown in figure 4, the untreated EDTA blood samples processed with the QIAzol (EDTA [-] QIAzol, EDTA c [-] QIAzol) and TDTMA protocol (EDTA [-] TDTMA) performed within expectations of unstabilized blood as gains of c-fos transcripts and losses of IL-1beta and p53 transcripts were observed. Blood samples drawn into PAXgene Blood RNA Tubes and processed with the PAX P protocol (PAX T [-] PAX P) performed within expectations for stabilized blood as constant levels of all three target transcripts were observed. These data verified the usefulness of the general experimental setup and approach as well as the validity of data sets. Solution A was the only candidate of test solutions that consistently stabilized the transcript levels of all genes analysed (EDTA [A] TDTMA).

As shown in figure 5, the untreated EDTA blood samples processed with the QIAzol (EDTA c [-] QIAzol) and TDTMA protocol (EDTA [-] TDTMA) verified the findings of unstabilized blood of the experiment shown in figure 4 as gains of c-fos transcripts and losses of IL-1beta transcripts were observed. Blood samples drawn into PAXgene Blood RNA Tubes and processed with the PAX P protocol (PAX T [-] PAX P) performed within expectations for stabilized blood as constant levels of both target transcripts were observed. These data verified again the validity of data sets and confirmed the findings of the experiment shown in figure 4. EDTA blood samples treated with test solution A (EDTA [A] TDTMA) showed stabilized transcript levels of c-fos and IL-1 beta with all donors.

The results of this example are also shown in figure 6. As shown in figure 6, the reference sample of cell stabilization ([+] control [EDTA]) showed the typical distribution of different subpopulations of WBC after storage for one day at room temperature. Lymphocytes, monocytes and granulocytes/neutrophils clustered differently and were distinguishable from debris in the sample. The negative control samples of cell stabilization ([-] control [EDTA + a.dest], [-] control [PAXgene]) showed complete lysis of all cells as expected, indicated by signals of smaller size and granularity within a cloud of data points without separation into different cell populations. All EDTA blood samples treated with test solutions (A-E) showed differentiation into cellular subpopulations, therefore indicating no lysis but integrity of cells.

The results demonstrate that isolation and purification of RNA from EDTA blood samples treated with different test solutions, especially with formaldehyde releaser diazolidinyl urea (DU) can be achieved with the method according to the present invention (TDTMA protocol). RNA prepared with this protocol is suitable to be analysed with both, monoplex and duplex RT-PCR assays. Transcripts in EDTA blood samples are stabilized when samples are treated with DU, at which DU does not destroy cell integrity.

## Example 5

As the method according to the present invention did result in sufficient and pure RNA suitable for RT-PCR analysis from blood samples treated with formaldehyde releaser (TDTMA protocol with STRECK samples and EDTA samples treated with DU) while other methods failed (see examples 1-4 for details), a variation of the invented method and the AllPrep DNA/RNA Mini protocol as suggested in US 2011/0111410 A1 (Ryan et al.) was investigated in terms of performance.

Peripheral blood samples from six donors were directly drawn into multiple BD EDTA tubes. Replicates of tubes were mixed with test solutions A and B within short time (maximum of 15 minutes) post collection and treated blood was directly contacted with the lysis composition (t0 samples) or after incubation for one day at room temperature (t24 samples), followed by RNA preparation with the protocols described below.

Two chemical formulations were selected that contain at least the formaldehyde releasing agent, known to require the method according to the present invention to prepare RNA. One volume of the following test solutions were mixed with 50 volumes of whole blood (200µl test solution added to 10mL of blood):

(A) 25% w/v diazolidinyl urea (DU)
(B) 25% w/v diazolidinyl urea (DU), 1.5 % (w/v) aurintricarboxylic acid, 0.8 % (w/v) sodium fluoride, 10 % (w/v) EDTA, 4.5 % (w/v) glyceraldehyde

RNA isolation and purification was performed using three different protocols:

(1) AllPrep: As the QIAGEN AllPrep DNA/RNA Mini Kit kit is intended for animal cells or tissue samples, but not for whole blood samples, WBC were isolated before use of the kit protocol. Therefore the AllPrep protocol combined a red blood cell lysis protocol and the use of the QIAGEN AllPrep DNA/RNA Mini Kit. In detail, aliquots of 0.75 ml of treated blood were mixed with 3.75 ml of erythrocyte lysis buffer EL (QIAGEN), incubated for 15 minutes on ice and centrifuged for 10 minutes at 400 rpm at 4°C to collect WBC. The WBC pellet was resuspended in 1.5 ml buffer EL and centrifuged again as described before. The RNA from the WBC pellet was isolated with the QIAGEN AllPrep DNA/RNA Mini Kit according to the handbook (November 2005). In brief, the cells were lyzed by adding 600 $\mu$l of buffer RLT Plus. Samples were homogenized via centrifugation through a QIAshredder spin column and DNA was removed by centrifugation of the lysate through the AllPrep DNA Mini Spin Column. RNA was bound to the RNeasy Mini Spin Column after addition of 600 $\mu$l 70% v/v ethanol. After washing steps with buffer RW1 and RPE, RNA was eluted from the spin column using RNAase-free water.

(2) TTAB - Method according to the present invention: Aliquots of 2.5 ml blood each were contacted after the indicated incubation times (t0, t24) with 7 ml of a lysis composition comprising a cationic detergent (here: 5 % w/v tetradecyltrimethylammonium bromide [TTAB] dissolved in 10 mM Tris buffer). The mixture was incubated at room temperature for 6 hours. The lysed samples were stored at -20 °C upon batchwise processing. After thawing, mixtures were centrifuged to harvest complexes of nucleic acids and detergents. Resulting pellets were processed further with the PreAnalytiX PAXgene Blood RNA Kit according to the kit handbook (Version 2, April 2008).

(3) TDTMA - Method according to the present invention: The TDTMA protocol described in example 3 was applied.

The yield and purity of the prepared RNA was determined from RNA aliquots as described in example 1. The results of this example are shown in figure 7.

The results show that the AllPrep protocol generated almost no RNA from all samples (on average 0.1 $\mu$g RNA/ ml blood), compared to samples processed with the TTAB and the TDTMA protocol that generated sufficient yields: on average 2.8 and 2.9 $\mu$g RNA/ ml blood, respectively.

RNA purity was in the typical range of highly pure RNA for all samples processed with the TTAB (24/24 samples; 100%) and the TDTMA protocol (24/24 samples; 100%), while the majority of AllPrep samples failed to demonstrate high purity (21/24 samples; 87.5% of samples were outside 1.8-2.2). Due to the low absorption values of the AllPrep samples, the determination of the purity was not reliable.

The results demonstrate that the two cationic detergent based protocols according to the present invention allow the isolation and purification of RNA from formaldehyde releaser treated blood samples by contacting the stabilized blood with a lysis composition comprising a cationic detergent and subsequent RNA preparation with the PAXgene Blood RNA Kit. Use of these protocols (TTAB protocol, TDTMA protocol) results in typical amounts of pure RNA, while the AllPrep protocol is inefficient to prepare pure RNA from stabilized blood samples.

**Claims**

1. A method for isolating nucleic acids from a stabilized sample or fraction thereof, wherein the sample stabilization involved the use of at least one formaldehyde releaser
**characterized in that** isolating the nucleic acids from the stabilized sample or fraction thereof involves the use of at least one cationic detergent during lysis of the stabilized sample or fraction thereof.

2. The method according to claim 1, **wherein** the cationic detergent is selected from the following group of cationic detergents:

   a) a cationic compound of the general formula (1):

   $$Y^+R_1R_2R_3R_4X^- \qquad (1)$$

   wherein

   Y represents nitrogen or phosphorus, preferably nitrogen
   $R_1, R_2, R_3$ and $R_4$ independently, represent a branched or unbranched $C_1$-$C_{20}$-alkyl group, a $C_6$-$C_{20}$-aryl group or a $C_6$-$C_{26}$ aralkyl group;
   $X^-$ represents an anion of an inorganic or organic, mono- or polybasic acid;

   b) a detergent comprising under the used lysis conditions a charged quaternary ammonium cation as polar head group;

c) a cationic detergent obtained in a composition comprising

(i) an amino surfactant having the following formula (2):

$$R1R2R3N(O)x \qquad (2)$$

wherein-

R1 and R2 each independently is H, C1-C20 alkyl residue, C6-C26 aryl residue or C6-C26 aralkyl residue, preferably H, C1-C6 alkyl residue, C6-C12 aryl residue or C6-C12 aralkyl residue,
R3 is C1 - C20 alkyl group, C6-C26 aryl residue or C6-C26 aralkyl residue,
X is an integer of 0 and 1 and

(ii) an acid or acid salt;

d) a cationic detergent obtained from an amino surfactant selected from the group consisting of the protonated forms of dodecylamine, N-methyldodecylamine, N, N-dimethyldodecylamine, N, N-dimethyldodecylamine N-oxide and 4-tetradecylaniline;

e) a cationic detergent comprising a permanently charged quaternary ammonium cation as polar head group, preferably an alkyltrimethylammonium salt; and/or

f) a cationic detergent selected from the group consisting of cetyl trimethyl ammonium bromide (CTAB), tetra decyl trimethyl ammonium bromide (TTAB) and dodecyl trimethyl ammonium bromide (DTRB) or the corresponding compounds comprising a chloride instead of the bromide.

3. The method according to claim 1, **wherein** the stabilized sample or fraction thereof is contacted with

a) a lysis composition comprising

(i) a cationic compound of the general formula (1):

$$Y^+R_1R_2R_3R_4X^- \qquad (1)$$

wherein Y represents nitrogen or phosphorus, preferably nitrogen
$R_1, R_2, R_3$ and $R_4$ independently, represent a branched or unbranched $C_1$-$C_{20}$-alkyl group, a $C_6$-$C_{20}$-aryl group or a $C_6$-$C_{26}$ aralkyl group;
$X^-$ represents an anion of an inorganic or organic, mono- or polybasic acid; and

(ii) at least one proton donor;
or

b) a lysis composition comprising

(i) an amino surfactant having the following formula (2):

$$R1R2R3N(O)x \qquad (2)$$

wherein,

R1 and R2 each independently is H, C1-C6 alkyl residue, C6-C12 aryl residue or C6-C12 aralkyl residue,
R3 is C1 - C20 alkyl group, C6-C26 aryl residue or C6-C26 aralkyl residue,
X is an integer of 0 and 1 and

(ii) an acid or acid salt.

4. The method according to claim 1, 2 or 3, comprising at least the following steps:

a. obtaining the stabilized sample or fraction thereof;
b. contacting the stabilized sample or fraction thereof with at least one cationic detergent for lysis and providing a lysed sample; and
c. isolating nucleic acids.

5. The method according to claim 4, comprising at least the following steps:

a. obtaining cells from the stabilized sample;
b. contacting the cells with at least one cationic detergent for lysis and providing a lysed sample; and
c. isolating nucleic acids.

6. The method according to claim 4 or 5, **wherein** step b. has one or more of the following characteristics:

i) the composition comprising the stabilized sample or fraction of the stabilized sample, the at least one cationic detergent and optionally one or more further lysis agents is incubated to provide the lysed sample;
ii) a nucleic acid containing portion is obtained from the lysed sample, preferably by sedimentation, and said nucleic acid containing portion is subjected to the nucleic acid isolation step c.; and/or
iii) the concentration of the cationic detergent in the lysis composition that is obtained when contacting the stabilized sample or fraction thereof with the cationic detergent and optionally one or more further lysis agents is selected from a range of 0.25% (w/v) to 30% (w/v), preferably 0.5% (w/v) to 15% (w/v).

7. The method according to one or more of claims 4 to 6, **wherein** step c. has one or more of the following characteristics:

i) nucleic acids are isolated from the lysed sample or from a nucleic acid containing portion obtained from the lysed sample;
ii) the lysed sample or the nucleic acid containing portion obtained from the lysed sample is contacted with one or more additional lysing agents thereby providing a lysis mixture;
iii) the nucleic acid isolation involves the use of a nucleic acid binding solid phase; and/or
iv) the nucleic acid isolation involves the use of at least one chaotropic salt and/or alcohol.

8. The method according to one or more of claims 4 to 7, wherein step c. comprises the following steps:

i) contacting the lysed sample or a nucleic acid containing portion obtained from the lysed sample with (aa) at least one chaotropic agent, preferably a chaotropic salt; (bb) at least one proteolytic enzyme, preferably proteinase K; and/or (cc) one or more salts, thereby providing a lysis mixture;
ii) binding nucleic acids contained in the lysis mixture to a nucleic acid binding solid phase, wherein in step ii) optionally the binding conditions are adjusted by adding a binding composition, wherein said binding composition preferably comprises a chaotropic salt and/or alcohol;
iii) separating the solid phase with the bound nucleic acids from the remaining sample; and
iv) optionally washing the nucleic acids and
v) optionally eluting nucleic acids from the solid phase.

9. The method according to one or more of claims 1 to 8, wherein the formaldehyde releaser used for stabilization of the sample has one or more of the following characteristics:

a) the formaldehyde releaser is selected from the group consisting of diazolidinyl urea, imidazolidinyl urea, dimethoylol-5,5dimethylhydantoin, dimethylol urea, 2-bromo-2.- nitropropane- 1,3-diol, oxazolidines, sodium hydroxymethyl glycinate, 5-hydroxymethoxymethyl-1 -1 aza-3,7-dioxabicyclo [3.3.0]octane, 5-hydroxymethyl-1 - 1 aza-3,7dioxabicyclo[3.3.0]octane, 5-hydroxypoly[methyleneoxy]methyl-1 -1 aza-3, 7dioxabicyclo[3.3.0]octane, quaternary adamantine and any combination of the foregoing;
b) the formaldehyde releaser is a heterocyclic urea, diazolidinyl urea and/or imidazolidinyl urea; and/or
c) the formaldehyde releaser is diazolidinyl urea.

10. The method according to one or more of claims 1 to 9, **wherein** the sample was stabilized additionally using one or more of the following or wherein the sample was stabilized using a stabilization composition additionally comprising one or more of the following:

a) one or more enzyme inhibitors, wherein said enzyme inhibitor has one or more of the following characteristics:

i) the enzyme inhibitor is a nuclease inhibitor;
ii) the enzyme inhibitor is selected from the group consisting of: dithiothreitol (DTT), iodoacetamide, iodoacetic acid, heparin, chitosan, cobalt chloride, diethyl pyrocarbonate, ethanol, aurintricarboxylic acid (ATA), glyceraldehydes, sodium fluoride, ethylenediamine tetraacetic acid (EDTA), formamide, vanadyl-ribonucleoside complexes, macaloid, hydroxylamine-oxygen- cupric ion, bentonite, ammonium sulfate, beta-mercaptoethanol, cysteine, dithioerythritol, tris (2-carboxyethyl) phosphene hydrochloride, a divalent cation such as $Mg^{2+}$, $Mn^{+2}$, $Zn^{+2}$, $Fe^{+2}$, $Ca^{2+}$, $Cu^{+2}$, and any combination of the foregoing; and/or
iii) the enzyme inhibitor is aurintricarboxylic acid;

b) one or more metabolic inhibitors having one or more of the following characteristics:

i) the metabolic inhibitor is selected from the group consisting of: dihydroxyacetone phosphate, glyceraldehyde 3-phosphate, 1,3-bisphosphoglycerate, 3-phosphoglycerate, 2- phosphoglycerate, phosphoenolpyruvate, pyruvate and glycerate dihydroxyacetate, sodium fluoride, $K_2C_2O_4$ and any combination of the foregoing;
ii) the metabolic inhibitor is glyceraldehyde;
iii) the metabolic inhibitor is sodium fluoride; and/or
iv) the metabolic inhibitor is a combination of glyceraldehyde and sodium fluoride;

c) one or more metal ion chelators wherein the metal ion chelator is selected from the group consisting of ethylene glycol tetraacetic acid (EGTA), 1 ,2-bis-(o-Aminophenoxy)-ethane-N',N',- N',N'-tetraacetic acid tetraacetoxy-Methyl ester (BAPTA-AM), dietyldithiocarbamate (DEDTC), ethylenediaminetetraacetic acid (EDTA), dicarboxymethyl- glutamic acid, nitrilotriacetic acid (NTA), ethylenediaminedisuccinic acid (EDDS), EDTA, citrat and any combination of the foregoing, preferably the metal chelator is EDTA.

11. The method according to one or more of claims 1 to 10, wherein the sample was stabilized using a stabilization composition comprising

a) a formaldehyde releaser agent selected from a chemical fixative that contains urea, preferably diazolidinyl urea and/or imidazolidinyl urea;
and at least one, two or preferably all of the following components:
b) an enzyme inhibitor;
c) a metabolic inhibitor, preferably glyceraldehyde and/or sodium fluoride;
d) a metal ion chelator, preferably EDTA.

12. The method according to one or more of claims 1 to 11, **wherein** the sample or fraction thereof has one or more of the following characteristics:

a) it comprises cells;
b) it is selected from the group consisting of whole blood, plasma, serum, lymphatic fluid, urine, liquor, ascites, milk, stool, bronchial lavage, saliva, bone marrow aspirates, amniotic fluid, semen/seminal fluid, swabs/smears, body fluids, body secretions, nasal secretions, vaginal secretions, wound secretions and excretions, cell suspensions, cell culture and cell culture supernatants;
c) it is a cell-free, cell-depleted or cell containing body fluid sample; and/or
d) it is whole blood.

13. The method according to one or more of claims 1 to 12, having one or more of the following characteristics:

a) the sample is mixed with the stabilization composition directly after and/or during the collection of the sample, thereby providing a stabilized sample;
b) cells are isolated from the stabilized sample and nucleic acids are isolated from the cells;
c) nucleic acids are isolated from a cell-free or cell-reduced portion of the stabilized sample; and/or
d) the isolated nucleic acids are in a further step processed and/or analyzed and preferably are:

i) modified;
ii) contacted with at least one enzyme;
iii) amplified;
iv) reverse transcribed;

v) cloned;

vi) sequenced;

vii) contacted with a probe; and/or

viii) detected;

and/or

e) the isolated nucleic acids are analyzed to identify, detect, screen for, monitor or exclude a disease or infection.

14. The method according to one or more of claims 1 to 13, wherein said method is for isolating RNA and wherein the sample is blood and wherein the blood stabilization involved the use of at least one formaldehyde releaser and at least one anticoagulant and wherein the method comprises the following steps:

a.) obtaining the stabilized blood sample or fraction thereof wherein the fraction of the stabilised blood sample is selected from blood cells, preferably white blood cells, serum or plasma;

b.) contacting the stabilized blood sample or fraction thereof with at least one cationic detergent and providing a lysed sample; and

c.) isolating nucleic acids, wherein said nucleic acids comprise or consist of RNA.

15. The method according to claim 14, wherein

- the formaldehyde releaser is selected from a heterocyclic urea, diazolidinyl urea and/or imidazolidinyl urea, and

- in step b) the cationic detergent is selected from the group defined in claim 2 or a lysis composition as defined in claim 3 is used and wherein the composition comprising the stabilized sample or fraction of the stabilized sample, the at least one cationic detergent and optionally one or more further lysis agents is incubated to provide the lysed sample; and

- step c) comprises the following steps:

i. contacting the lysed sample or a nucleic acid containing portion obtained from the lysed sample with one or more additional lysing agents thereby providing a lysis mixture, wherein preferably for this purpose the lysed sample or the nucleic acid containing portion obtained from the lysed sample is contacted with (aa) at least one chaotropic agent, preferably a chaotropic salt; (bb) at least one proteolytic enzyme; and/or (cc) one or more salts, thereby providing a lysis mixture;

- optionally removing DNA from the lysis mixture;

ii. adding alcohol to the lysis mixture to adjust the binding conditions and binding RNA to a nucleic acid binding solid phase;

iii. separating the solid phase with the bound RNA from the remaining sample; and

iv. optionally washing the RNA and

v. optionally eluting RNA from the solid phase.

**Patentansprüche**

1. Verfahren zur Isolierung von Nukleinsäuren aus einer stabilisierten Probe oder Fraktion davon, wobei die Probenstabilisierung die Verwendung von mindestens einem Formaldehyd-Releaser beinhaltete, **dadurch gekennzeichnet, dass** die Isolierung der Nukleinsäuren aus der stabilisierten Probe oder Fraktion davon die Verwendung von mindestens einem kationischen Detergenz während der Lyse der stabilisierten Probe oder Fraktion davon umfasst.

2. Das Verfahren nach Anspruch 1, **wobei** das kationische Detergenz ausgewählt ist aus der folgenden Gruppe von kationischen Detergenzien:

a) eine kationische Verbindung der allgemeinen Formel (1):

$$Y^+R_1R_2R_3R_4X^-\qquad(1)$$

wobei

Y Stickstoff oder Phosphor darstellt, vorzugsweise Stickstoff

$R_1$, $R_2$, $R_3$ und $R_4$ unabhängig voneinander eine verzweigte oder unverzweigte $C_1$-$C_{20}$-Alkyl-Gruppe, eine $C_6$-$C_{20}$-Aryl-Gruppe oder eine $C_6$-$C_{26}$-Aralkyl-Gruppe darstellen;

$X^-$ ein Anion einer anorganischen oder organischen, mono- oder polybasischen Säure darstellt;

b) ein Detergenz, das unter den eingesetzten Lyse-Bedingungen ein geladenes quaternäres Ammonium-Kation als polare Kopfgruppe aufweist;

c) ein kationisches Detergenz, das in einer Zusammensetzung erhalten wird, die umfasst

(i) ein Amino-Tensid mit der folgenden Formel (2):

$$R1R2R3N(O)x \qquad (2)$$

wobei

R1 und R2 jeweils unabhängig voneinander H, C1-C20-Alkyl-Rest, C6-C26-Aryl-Rest oder C6-C26-Aralkyl-Rest, vorzugsweise H, C1-C6 Alkyl-Rest, C6-C12-Aryl-Rest oder C6-C12-Aralkyl-Rest sind,
R3 eine C1-C20-Alkyl-Gruppe, ein C6-C26-Aryl-Rest oder C6-C26-Aralkyl-Rest ist,
X eine Ganzzahl von 0 und 1 ist und

(ii) eine Säure oder ein Säuresalz ist;

d) ein kationisches Detergenz, das von einem Amino-Tensid erhalten wird ausgewählt aus der Gruppe bestehend aus den protonierten Formen von Dodecylamin, N-Methyldodecylamin, N,N-Dimethyldodecylamin, N,N-Dimethyldodecylamin-N-oxid und 4-Tetradecylanilin;

e) ein kationisches Detergenz, das ein permanent geladenes quartarnäres Ammonium-Kation als polare Kopfgruppe aufweist, vorzugsweise ein Alkyltrimethylammonium-Salz; und/oder

f) ein kationisches Detergenz, ausgewählt aus der Gruppe bestehend aus Cetyltrimethylammoniumbromid (CTAB), Tetradecyltrimethylammoniumbromid (TTAB) und Dodecyltrimethylammoniumbromid (DTRB) oder die entsprechenden Verbindungen umfassend ein Chlorid anstelle von Bromid.

3. Das Verfahren nach Anspruch 1, **wobei** die stabilisierte Probe oder Fraktion davon in Kontakt gebracht wird mit

a) einer Lyse-Zusammensetzung, umfassend

(i) eine kationische Verbindung der allgemeinen Formel (1):

$$Y^+R_1R_2R_3R_4X^- \qquad (1)$$

wobei Y Stickstoff oder Phosphor darstellt, vorzugsweise Stickstoff
$R_1$, $R_2$, $R_3$ und $R_4$ unabhängig voneinander eine verzweigte oder unverzweigte $C_1$-$C_{20}$-Alkyl-Gruppe, eine $C_6$-$C_{20}$-Aryl-Gruppe oder eine $C_6$-$C_{26}$-Aralkyl-Gruppe darstellen;
$X^-$ ein Anion einer anorganischen oder organischen, mono- oder polybasischen Säure darstellt; und

(ii) mindestens einen Proton-Donator;
oder

b) eine Lyse-Zusammensetzung, umfassend

(i) ein Amino-Tensid gemäß der folgenden Formel (2):

$$R1R2R3N(O)x \qquad (2)$$

wobei

R1 und R2 jeweils unabhängig voneinander H, C1-C6-Alkyl-Rest, C6-C12-Aryl-Rest oder C6-C12-Aralkyl-Rest sind,

R3 eine C1-C20-Alkyl-Gruppe, ein C6-C26-Aryl-Rest oder ein C6-C26-Aralkyl-Rest ist,
X eine Ganzzahl von 0 und 1 und

(ii) eine Säure oder ein Säuresalz ist.

**4.** Das Verfahren nach Anspruch 1, 2 oder 3, umfassend mindestens die folgenden Schritte:

a. Erhalt der stabilisierten Probe oder Fraktion davon;
b. Inkontaktbringen der stabilisierten Probe oder Fraktion davon mit mindestens einem kationischen Detergenz zur Lyse und Bereitstellen einer lysierten Probe; und
c. Isolieren der Nukleinsäuren.

**5.** Das Verfahren nach Anspruch 4, umfassend mindestens die folgenden Schritte:

a. Erhalt von Zellen aus der stabilisierten Probe;
b. Inkontaktbringen der Zellen mit mindestens einem kationischen Detergenz zur Lyse und Bereitstellen einer lysierten Probe; und
c. Isolieren der Nukleinsäuren.

**6.** Das Verfahren nach Anspruch 4 oder 5, **wobei** Schritt b. ein oder mehrere der folgenden Merkmale aufweist:

i) die Zusammensetzung umfassend die stabilisierte Probe oder Fraktion der stabilisierten Probe, das mindestens eine kationische Detergenz und optional ein oder mehrere weitere Lyse-Mittel wird inkubiert um die lysierte Probe bereitzustellen;
ii) ein Nukleinsäure-enthaltender Anteil wird aus der lysierten Probe erhalten, vorzugsweise durch Sedimentierung, und der Nukleinsäure-enthaltende Anteil wird dem Nukleinsäure-Isolierungsschritt c. zugeführt; und/oder
iii) die Konzentration des kationischen Detergenz in der Lyse-Zusammensetzung, die beim Inkontaktbringen der stabilisierten Probe oder Fraktion davon mit dem kationischen Detergenz und optional einem oder mehreren weiteren Lyse-Mitteln erhalten wird, ist ausgewählt aus einem Bereich von 0,25 % (Gew./Vol.) bis 30 % (Gew./Vol.), vorzugsweise 0,5 % (Gew./Vol.) bis 15 % (Gew./Vol.).

**7.** Das Verfahren nach einem oder mehreren der Ansprüche 4 bis 6, **wobei** Schritt c. ein oder mehrere der folgenden Merkmale aufweist:

i) Nukleinsäuren werden aus der lysierten Probe oder aus einem Nukleinsäure-enthaltenden Anteil, der aus der lysierten Probe erhalten wurde, isoliert;
ii) die lysierte Probe oder der Nukleinsäure-enthaltende Anteil, der aus der lysierten Probe erhalten wurde, wird mit einem oder mehreren zusätzlichen Lyse-Mitteln in Kontakt gebracht, wodurch ein Lyse-Gemisch bereitgestellt wird;
iii) die Nukleinsäureisolierung umfasst die Verwendung einer Nukleinsäure-bindenden Festphase; und/oder
iv) die Nukleinsäureisolierung umfasst die Verwendung von mindestens einem chaotropen Salz und/oder Alkohol.

**8.** Das Verfahren nach einem oder mehreren der Ansprüche 4 bis 7, wobei Schritt c. die folgenden Schritte umfasst:

i) Inkontaktbringen der lysierten Probe oder eines Nukleinsäure-enthaltenden Anteils, der aus der lysierten Probe erhalten wurde, mit (aa) mindestens einem chaotropen Agens, vorzugsweise einem chaotropen Salz; (bb) mindestens einem proteolytischen Enzym, vorzugsweise Proteinase K; und/oder (cc) einem oder mehreren Salzen, wodurch ein Lyse-Gemisch bereitgestellt wird,
ii) Binden von Nukleinsäuren, die in dem Lyse-Gemisch enthalten sind, an eine Nukleinsäure-bindende Festphase, wobei in Schritt ii) optional die Bindungsbedingungen durch Hinzufügen einer Bindungszusammensetzung eingestellt werden, wobei die Bindungszusammensetzung vorzugsweise ein chaotropes Salz und/oder Alkohol umfasst;
iii) Separieren der Festphase mit den gebundenen Nukleinsäuren aus der verbleibenden Probe; und
iv) optional Waschen der Nukleinsäuren, und
v) optional Eluieren der Nukleinsäuren von der Festphase.

**9.** Das Verfahren nach einem oder mehreren der Ansprüche 1 bis 8, wobei der zur Stabilisierung der Probe eingesetzte Formaldehyd-Releaser ein oder mehrere der folgenden Merkmale aufweist:

a) der Formaldehyd-Releaser ist ausgewählt aus der Gruppe bestehend aus Diazolidinyl-Harnstoff, Imidazolidinyl-Harnstoff, Dimethylol-5,5-dimethylhydantoin, Dimethylol-Harnstoff, 2-Brom-2-nitropropan-1,3-diol, Oxazolidin, Natrium-Hydroxymethylglyzinat, 5-Hydroxymethoxymethyl-1-1-aza-3,7-dioxabicyclo[3.3.0]octan, 5-Hydroxymethyl-1-1-aza-3,7dioxabicyclo[3.3.0]octan, 5-Hydroxypoly[methylenoxy]methyl-1-1-aza-3,7dioxabicyclo[3.3.0]octan, quaternärem Adamantin und jeglicher Kombination davon;

b) der Formaldehyd-Releaser ist ein heterocyclischer Harnstoff, Diazolidinyl-Harnstoff und/oder Imidazolidinyl-Harnstoff; und/oder

c) der Formaldehyd-Releaser ist Diazolidinyl-Harnstoff.

10. Das Verfahren nach einem oder mehreren der Ansprüche 1 bis 9, **wobei** die Probe durch zusätzliche Verwendung von einem oder mehreren des Folgenden stabilisiert wurde, oder wobei die Probe unter Verwendung einer Stabilisierungszusammensetzung stabilisiert wurde, die zusätzlich eines oder mehrere des Folgenden umfasst:

a) ein oder mehrere Enzyminhibitoren, wobei der Enzyminhibitor ein oder mehrere der folgenden Merkmale aufweist:

i) der Enzyminhibitor ist ein Nuklease-Hemmer;

ii) der Enzyminhibitor ist ausgewählt aus der Gruppe bestehend aus: Dithiothreitol (DTT), Iodoacetamid, Jodessigsäure, Heparin, Chitosan, Kobaltchlorid, Diethylpyrocarbonat, Ethanol, Aurintricarbonsäure (ATA), Glycerinaldehyde, Natriumfluorid, Ethylenediamintetraessigsäure (EDTA), Formamid, Vanadyl-Ribonukleosid-Komplexe, Macaloid, Hydroxylamin-Sauerstoff-Kupfer-Ion, Bentonit, Ammoniumsulfat, beta-Mercaptoethanol, Cystein, Dithioerythritol, Tris(2-Carboxyethyl)phosphenhydrochlorid, einem divalentes Kation, wie z. B. $Mg^{2+}$, $Mn^{+2}$, $Zn^{+2}$, $Fe^{+2}$, $Ca^{2+}$, $Cu^{+2}$ und jeder Kombination der vorherigen; und/oder

iii) der Enzyminhibitor ist Aurintricarbonsäure;

b) ein oder mehrere metabolische Hemmer mit einem oder mehreren der folgenden Merkmale:

i) die metabolische Hemmer ist ausgewählt aus der Gruppe bestehend aus: Dihydroxyacetonphosphat, Glycerinaldehyd-3-phosphat, 1,3-Bisphosphoglycerat, 3-Phosphoglycerat, 2-Phosphoglycerat, Phosphoenolpyruvat, Pyruvat und Glyzeratdihydroxyacetat, Natriumfluorid, $K_2C_2O_4$ und jede Kombination der vorherigen;

ii) der metabolische Hemmer ist Glycerinaldehyd;

iii) der metabolische Hemmer ist Natriumfluorid; und/oder

iv) der metabolische Hemmer ist eine Kombination aus Glycerinaldehyd und Natriumfluorid;

c) ein oder mehrere Metallionen Chelatoren, wobei der Metallionen Chelator ausgewählt ist aus der Gruppe bestehend aus Ethylenglycoltetraessigsäure (EGTA), 1,2-bis-(o-Aminophenoxy)-ethan-N',N',-N',N'-tetraessigsäure Tetraacetoxy-Methylester (BAPTA-AM), Dietyldithiocarbamat (DEDTC), Ethylendiamintetraessigsäure (EDTA), Dicarboxymethylglutaminsäure, Nitrilotriessigsäure (NTA), Ethylendiamindisuccinylsäure (EDDS), EDTA, Citrat und jeder Kombination der vorherigen, vorzugsweise ist der Metall-Chelator EDTA.

11. Das Verfahren nach einem oder mehreren der Ansprüche 1 bis 10, wobei die Probe unter Verwendung einer Stabilisierungszusammensetzung stabilisiert wurde, umfassend

a) ein Formaldehyd-Releaser Agens, ausgewählt aus einem chemischen Fixativ, das Harnstoff enthält, vorzugsweise Diazolidinyl-Harnstoff und/oder Imidazolidinyl-Harnstoff;

und mindestens eine, zwei oder vorzugsweise alle der folgenden Komponenten:

b) einen Enzyminhibitor;

c) einen metabolischen Hemmer, vorzugsweise Glycerinaldehyd und/oder Natriumfluorid;

d) einen Metallionen Chelator, vorzugsweise EDTA.

12. Das Verfahren nach einem oder mehreren der Ansprüche 1 bis 11, **wobei** die Probe oder Fraktion davon ein oder mehrere der folgenden Merkmale aufweist:

a) sie umfasst Zellen;

b) sie ist ausgewählt aus der Gruppe bestehend aus Vollblut, Plasma, Serum, Lymphflüssigkeit, Urin, Liquor, Aszites, Milch, Stuhl, Bronchiallavage, Speichel, Knochenmarkaspirate, Fruchtwasser, Samen/Samenflüssigkeit, Abtupfer/Abstriche, Körperflüssigkeiten, Körpersekretionen, Nasensekrete, Vaginalsekrete, Wundsekrete und -Exkretionen, Zellsuspensionen, Zellkultur und Zellkultur-Überstände;

c) sie ist eine zellfreie, zell-abgereicherte oder zellhaltige Körperflüssigkeit-Probe; und/oder

d) sie ist Vollblut.

13. Das Verfahren nach einem oder mehreren der Ansprüche 1 bis 12, das ein oder mehrere der folgenden Merkmale aufweist:

a) die Probe wird mit der Stabilisierungszusammensetzung direkt nach und/oder während des Sammelns der Probe gemischt, wodurch eine stabilisierte Probe bereitgestellt wird;

b) Zellen werden aus der stabilisierten Probe isoliert und Nukleinsäuren werden aus den Zellen isoliert;

c) Nukleinsäuren werden aus einem zellfreien oder zellreduziertem Anteil der stabilisierten Probe isoliert; und/oder

d) die isolierten Nukleinsäuren werden in einem weiteren Schritt verarbeitet und/oder analysiert, und werden vorzugsweise:

i) modifiziert;

ii) mit mindestens einem Enzym in Kontakt gebracht;

iii) amplifiziert;

iv) revers transkribiert;

v) geklont;

vi) sequenziert;

vii) mit einer Sonde in Kontakt gebracht; und/oder

viii) detektiert; und/oder

e) die isolierten Nukleinsäuren werden analysiert, um eine Krankheit oder Infektion zu identifizieren, detektieren, screenen, überwachen oder auszuschließen.

14. Das Verfahren nach einem oder mehreren der Ansprüche 1 bis 13, wobei das Verfahren zur Isolierung von RNA ist und wobei die Probe Blut ist, und wobei die Blutstabilisierung die Verwendung von mindestens einem Formaldehyd-Releaser und mindestens einem Antikoagulans involvierte, und wobei das Verfahren die folgenden Schritte umfasst:

a.) Erhalt der stabilisierten Blutprobe oder Fraktion davon, wobei die Fraktion der stabilisierten Blutprobe ausgewählt ist aus Blutzellen, vorzugsweise weißen Blutzellen, Serum oder Plasma;

b.) Inkontaktbringen der stabilisierten Blutprobe oder Fraktion davon mit mindestens einem kationischen Detergenz und Bereitstellen einer lysierten Probe; und

c.) Isolieren der Nukleinsäuren, wobei die Nukleinsäuren RNA aufweisen oder daraus bestehen.

15. Das Verfahren nach Anspruch 14, wobei

- der Formaldehyd-Releaser ausgewählt ist aus einem heterocyclischen Harnstoff, Diazolidinyl-Harnstoff und/oder Imidazolidinyl-Harnstoff, und

- in Schritt b) das kationische Detergenz ausgewählt ist aus der in Anspruch 2 definierten Gruppe, oder eine Lyse-Zusammensetzung wie in Anspruch 3 definiert verwendet wird, und wobei die Zusammensetzung umfassend die stabilisierte Probe oder Fraktion der stabilisierten Probe, das mindestens eine kationische Detergenz und optional das eine oder die mehreren weiteren Lyse-Mittel inkubiert wird, um die lysierte Probe bereitzustellen; und

- Schritt c) die folgenden Schritte umfasst:

i. Inkontaktbringen der lysierten Probe oder eines Nukleinsäure-enthaltenden Anteils, der aus der lysierten Probe erhalten wurde, mit einem oder mehreren zusätzlichen Lyse-Mitteln, wodurch ein Lyse-Gemisch bereitgestellt wird, wobei vorzugsweise zu diesem Zweck die lysierte Probe oder der Nukleinsäure-enthaltende Anteil, der aus der lysierten Probe erhalten wurde, mit (aa) mindestens einem chaotropen Agens, vorzugsweise einem chaotropen Salz; (bb) mindestens einem proteolytischen Enzym; und/oder (cc) einem oder mehreren Salzen in Kontakt gebracht wird, wodurch ein Lyse-Gemisch bereitgestellt wird;

- optional Entfernen von DNA aus dem Lyse-Gemisch;

ii. Hinzufügen von Alkohol zu dem Lyse-Gemisch, um die Bindungsbedingungen einzustellen und Binden von RNA an eine Nukleinsäure-bindende Fetsphase;
iii. Separieren der Festphase mit der gebundenen RNA aus der verbleibenden Probe; und
iv. optional Waschen der RNA und
v. optional Eluieren von RNA von der Festphase.

**Revendications**

1. Procédé pour isoler des acides nucléiques à partir d'un échantillon stabilisé ou d'une fraction de celui-ci, où la stabilisation de l'échantillon a impliqué l'utilisation d'au moins un libérateur de formaldéhyde **caractérisé en ce que** l'isolement des acides nucléiques à partir de l'échantillon stabilisé ou d'une fraction de celui-ci implique l'utilisation d'au moins un détergent cationique pendant la lyse de l'échantillon stabilisé ou d'une fraction de celui-ci.

2. Procédé selon la revendication 1, **dans lequel** le détergent cationique est sélectionné parmi le groupe suivant de détergents cationiques :

a) un composé cationique de la formule générale (1) :

$$Y^+R_1R_2R_3R_4X^- \qquad (1)$$

où

Y représente un azote ou un phosphore, de préférence un azote
$R_1$, $R_2$, $R_3$ et $R_4$ représentent indépendamment un groupement alkyle en $C_1$ à $C_{20}$ ramifié ou non ramifié, un groupement aryle en $C_6$ à $C_{20}$ ou un groupement aralkyle en $C_6$ à $C_{26}$ ;
$X^-$ représente un anion d'un monoacide ou d'un polyacide, inorganique ou organique ;

b) un détergent comprenant dans les conditions de lyse utilisées un cation ammonium quaternaire chargé en tant que groupement de tête polaire ;
c) un détergent cationique obtenu dans une composition comprenant (i) un tensioactif aminé présentant la formule suivante (2) :

$$R1R2R3N(O)x \qquad (2)$$

où

R1 et R2 représentent chacun indépendamment un H, un résidu alkyle en C1 à C20, un résidu aryle en C6 à C26 ou un résidu aralkyle en C6 à C26, de préférence un H, un résidu alkyle en C1 à C6, un résidu aryle en C6 à C12 ou un résidu aralkyle en C6 à C12,
R3 est un groupement alkyle en C1 à C20, un résidu aryle en C6 à C26 ou un résidu aralkyle en C6 à C26,
X est un entier parmi 0 et 1 et

(ii) un acide ou un sel acide ;

d) un détergent cationique obtenu à partir d'un tensioactif aminé sélectionné parmi le groupe constitué des formes protonées de dodécylamine, de N-méthyldodécylamine, de N,N-diméthyldodécylamine, de N-oxyde de N,N-diméthyldodécylamine et de 4-tétradécylaniline ;
e) un détergent cationique comprenant un cation ammonium quaternaire chargé en permanence en tant que groupement de tête polaire, de préférence un sel d'alkyltriméthylammonium ; et/ou
f) un détergent cationique sélectionné parmi le groupe constitué de bromure de cétyltriméthylammonium (CTAB), de bromure de tétradécyltriméthylammonium (TTAB) et de bromure de dodécyltriméthylammonium (DTRB) ou les composés correspondants comprenant un chlorure à la place du bromure.

3. Procédé selon la revendication 1, **dans lequel** l'échantillon stabilisé ou une fraction de celui-ci est mis en contact avec

a) une composition de lyse comprenant

(i) un composé cationique de la formule générale (1) :

$$Y^+R_1R_2R_3R_aX^- \qquad (1)$$

où Y représente un azote ou un phosphore, de préférence un azote
$R_1$, $R_2$, $R_3$ et $R_4$ représentent indépendamment un groupement alkyle en $C_1$ à $C_{20}$ ramifié ou non ramifié, un groupement aryle en $C_6$ à $C_{20}$ ou un groupement aralkyle en $C_6$ à $C_{26}$ ;
$X^-$ représente un anion d'un monoacide ou d'un polyacide, inorganique ou organique ; et

(ii) au moins un donneur de proton ;ou

b) une composition de lyse comprenant

(i) un tensioactif aminé présentant la formule suivante (2) :

$$R1R2R3N(O)x \qquad (2)$$

où

R1 et R2 représentent chacun indépendamment un H, un résidu alkyle en C1 à C6, un résidu aryle en C6 à C12 ou un résidu aralkyle en C6 à C12,
R3 est un groupement alkyle en C1 à C20, un résidu aryle en C6 à C26 ou un résidu aralkyle en C6 à C26,
X est un entier parmi 0 et 1 et

(ii) un acide ou un sel acide.

4. Procédé selon la revendication 1, 2 ou 3, comprenant au moins les étapes suivantes :

a. obtenir l'échantillon stabilisé ou une fraction de celui-ci ;
b. mettre en contact l'échantillon stabilisé ou une fraction de celui-ci avec au moins un détergent cationique pour la lyse et fournir un échantillon lysé ; et
c. isoler des acides nucléiques.

5. Procédé selon la revendication 4, comprenant au moins les étapes suivantes :

a. obtenir des cellules à partir de l'échantillon stabilisé ;
b. mettre en contact les cellules avec au moins un détergent cationique pour la lyse et fournir un échantillon lysé ; et
c. isoler des acides nucléiques.

6. Procédé selon la revendication 4 ou 5, **dans lequel** l'étape b. présente une ou plusieurs des caractéristiques suivantes :

i) la composition comprenant l'échantillon stabilisé ou une fraction de l'échantillon stabilisé, le au moins un détergent cationique et optionnellement un ou plusieurs autres agents de lyse est incubée pour fournir l'échantillon lysé ;
ii) une portion contenant des acides nucléiques est obtenue à partir de l'échantillon lysé, de préférence par sédimentation, et ladite portion contenant des acides nucléiques est soumise à l'étape c. d'isolement d'acides nucléiques ; et/ou
iii) la concentration du détergent cationique dans la composition de lyse qui est obtenue lors de la mise en contact de l'échantillon stabilisé ou d'une fraction de celui-ci avec le détergent cationique et optionnellement un ou plusieurs autres agents de lyse est sélectionnée dans une plage allant de 0,25 % (m/v) à 30 % (m/v), de préférence allant de 0,5 % (m/v) à 15 % (m/v).

7. Procédé selon une ou plusieurs des revendications 4 à 6, **dans lequel** l'étape c. présente une ou plusieurs des caractéristiques suivantes :

i) des acides nucléiques sont isolés à partir de l'échantillon lysé ou à partir d'une portion contenant des acides nucléiques obtenue à partir de l'échantillon lysé ;

ii) l'échantillon lysé ou la portion contenant des acides nucléiques obtenue à partir de l'échantillon lysé est mis en contact avec un ou plusieurs agents de lyse supplémentaires en fournissant ainsi un mélange de lyse ;

iii) l'isolement des acides nucléiques implique l'utilisation d'une phase solide de liaison d'acide nucléique ; et/ou

iv) l'isolement des acides nucléiques implique l'utilisation d'au moins un sel et/ou alcool chaotropique.

8. Procédé selon une ou plusieurs des revendications 4 à 7, dans lequel l'étape c. comprend les étapes suivantes :

i) mettre en contact l'échantillon lysé ou une portion contenant des acides nucléiques obtenue à partir de l'échantillon lysé avec (aa) au moins un agent chaotropique, de préférence un sel chaotropique ; (bb) au moins une enzyme protéolytique, de préférence une protéinase K ; et/ou (cc) un ou plusieurs sels, en fournissant ainsi un mélange de lyse ;

ii) lier des acides nucléiques contenus dans le mélange de lyse à une phase solide de liaison d'acide nucléique, où lors de l'étape ii), optionnellement les conditions de liaison sont ajustées en ajoutant une composition de liaison, où ladite composition de liaison comprend de préférence un sel et/ou alcool chaotropique ;

iii) séparer la phase solide avec les acides nucléiques liés du reste de l'échantillon ; et

iv) optionnellement laver les acides nucléiques et

v) optionnellement éluer des acides nucléiques de la phase solide.

9. Procédé selon une ou plusieurs des revendications 1 à 8, dans lequel le libérateur de formaldéhyde utilisé pour la stabilisation de l'échantillon présente une ou plusieurs des caractéristiques suivantes :

a) le libérateur de formaldéhyde est sélectionné parmi le groupe constitué de la diazolidinyl-urée, de l'imidazolidinyl-urée, de la diméthoylol-5,5-diméthylhydantoïne, de la diméthylol-urée, du 2-bromo-2-nitropropane-1,3-diol, d'oxazolidines, de l'hydroxyméthylglycinate de sodium, du 5-hydroxyméthoxyméthyl-1-1-aza-3,7-dioxabicyclo[3.3.0]octane, du 5-hydroxyméthyl-1-1-aza-3,7-dioxabicyclo[3.3.0]octane, du 5-hydroxypoly[méthylèneoxy]méthyl-1-1-aza-3,7-dioxabicyclo[3.3.0]octane, de l'adamantine quaternaire et de toute combinaison de ceux-ci ;

b) le libérateur de formaldéhyde est une urée hétérocyclique, la diazolidinyl-urée et/ou l'imidazolidinyl-urée ; et/ou

c) le libérateur de formaldéhyde est la diazolidinyl-urée.

10. Procédé selon une ou plusieurs des revendications 1 à 9, **dans lequel** l'échantillon a été stabilisé en utilisant en plus un ou plusieurs des composés suivants ou dans lequel l'échantillon a été stabilisé en utilisant une composition de stabilisation comprenant en plus un ou plusieurs des composés suivants :

a) un ou plusieurs inhibiteurs d'enzyme, où ledit inhibiteur d'enzyme présente une ou plusieurs des caractéristiques suivantes :

i) l'inhibiteur d'enzyme est un inhibiteur de nucléase ;

ii) l'inhibiteur d'enzyme est sélectionné parmi le groupe constitué : du dithiothréitol (DTT), de l'iodoacétamide, de l'acide iodoacétique, de l'héparine, du chitosan, du chlorure de cobalt, du pyrocarbonate de diéthyle, de l'éthanol, de l'acide aurintricarboxylique (ATA), de glycéraldéhydes, du fluorure de sodium, de l'acide éthylène diamine tétraacétique (EDTA), du formamide, de complexes de vanadyle-ribonucléoside, du macaloïde, de l'ion cuprique-oxygène-hydroxylamine, de la bentonite, du sulfate d'ammonium, du bêta-mercaptoéthanol, de la cystéine, du dithioérythritol, de l'hydrochlorure de tris(2-carboxyéthyle)-phopshène, d'un cation divalent tel que $Mg^{2+}$, $Mn^{2+}$, $Zn^{2+}$, $Fe^{2+}$, $Ca^{2+}$, $Cu^{2+}$, et de toute combinaison de ceux-ci ; et/ou

iii) l'inhibiteur d'enzyme est l'acide aurintricarboxylique ;

b) un ou plusieurs inhibiteurs métaboliques présentant une ou plusieurs des caractéristiques suivantes :

i) l'inhibiteur métabolique est sélectionné parmi le groupe constitué : de la dihydroxyacétone phosphate, du glycéraldéhyde-3-phosphate, du 1,3-bisphosphoglycérate, du 3-phosphoglycérate, du 2-phosphoglycérate, du phosphoénolpyruvate, du dihydroxyacétate de pyruvate et de glycérate, du fluorure de sodium, du $K_2C_2O_4$ et de toute combinaison de ceux-ci ;

ii) l'inhibiteur métabolique est du glycéraldéhyde ;

iii) l'inhibiteur métabolique est du fluorure de sodium ; et/ou

iv) l'inhibiteur métabolique est une combinaison de glycéraldéhyde et de fluorure de sodium ;

c) un ou plusieurs chélateurs d'ion métallique où le chélateur d'ion métallique est sélectionné parmi le groupe constitué de l'acide éthylène glycol tétraacétique (EGTA), de l'acide 1,2-bis-(o-aminophénoxy)-éthane-N',N',N',N'-tétraacétique-ester tétraacétoxyméthylique (BAPTA-AM), du diéthyldithiocarbamate (DEDTC), de l'acide éthylène diamine tétraacétique (EDTA), de l'acide dicarboxyméthylglutamique, de l'acide nitrilotriacétique (NTA), de l'acide éthylènediaminedisuccinique (EDDS), de l'EDTA, d'un citrate et de toute combinaison de ceux-ci, de préférence le chélateur de métal est l'EDTA.

**11.** Procédé selon une ou plusieurs des revendications 1 à 10, dans lequel l'échantillon a été stabilisé en utilisant une composition de stabilisation comprenant

a) un agent libérateur de formaldéhyde sélectionné à partir d'un fixateur chimique qui contient une urée, de préférence la diazolidinyl-urée et/ou l'imidazolidinyl-urée ;
et au moins un, deux ou de préférence la totalité des composés suivants :
b) un inhibiteur d'enzyme ;
c) un inhibiteur métabolique, de préférence du glycéraldéhyde et/ou du fluorure de sodium ;
d) un chélateur d'ion métallique, de préférence l'EDTA.

**12.** Procédé selon une ou plusieurs des revendications 1 à 11, **dans lequel** l'échantillon ou une fraction de celui-ci présente une ou plusieurs des caractéristiques suivantes :

a) il comprend des cellules ;
b) il est sélectionné parmi le groupe constitué de sang entier, de plasma, de sérum, de fluide lymphatique, d'urine, de liqueur, d'ascites, de lait, de selles, de lavage bronchial, de salive, de ponctions de moelle osseuse, de fluide amniotique, de sperme/fluide séminal, d'écouvillons/de frottis, de fluides corporels, de sécrétions corporelles, de sécrétions nasales, de sécrétions vaginales, de sécrétions et d'excrétions de plaie, de suspensions cellulaires, de culture cellulaire et de surnageants de culture cellulaire ;
c) il s'agit d'un échantillon de fluide corporel exempt de cellules, appauvri en cellules ou contenant des cellules ; et/ou
d) il s'agit de sang entier.

**13.** Procédé selon une ou plusieurs des revendications 1 à 12, présentant une ou plusieurs des caractéristiques suivantes :

a) l'échantillon est mélangé avec la composition de stabilisation juste après et/ou pendant la collecte de l'échantillon, en fournissant ainsi un échantillon stabilisé ;
b) des cellules sont isolées à partir de l'échantillon stabilisé et des acides nucléiques sont isolés à partir des cellules ;
c) des acides nucléiques sont isolés à partir d'une portion exempte de cellules ou appauvrie en cellules de l'échantillon stabilisé ; et/ou
d) les acides nucléiques isolés sont traités dans une autre étape et/ou analysés et de préférence sont :

i) modifiés ;
ii) mis en contact avec au moins une enzyme ;
iii) amplifiés ;
iv) transformés par transcription inverse ;
v) clonés ;
vi) séquencés ;
vii) mis en contact avec une sonde ; et/ou
viii) détectés ;et/ou

e) les acides nucléiques isolés sont analysés pour identifier, détecter, dépister, surveiller ou exclure une maladie ou une infection.

**14.** Procédé selon une ou plusieurs des revendications 1 à 13, où ledit procédé est destiné à isoler de l'ARN et où l'échantillon est du sang et où la stabilisation du sang a impliqué l'utilisation d'au moins un libérateur de formaldéhyde et d'au moins un anticoagulant et où le procédé comprend les étapes suivantes :

a.) obtenir l'échantillon de sang stabilisé ou une fraction de celui-ci, où la fraction de l'échantillon de sang stabilisé est sélectionnée parmi les cellules sanguines, de préférence les globules blancs, le sérum ou le plasma ;

b.) mettre en contact l'échantillon de sang stabilisé ou une fraction de celui-ci avec au moins un détergent cationique et fournir un échantillon lysé ; et

c.) isoler des acides nucléiques, où lesdits acides nucléiques comprennent ou se composent d'ARN.

**15.** Procédé selon la revendication 14, dans lequel

- le libérateur de formaldéhyde est sélectionnée parmi une urée hétérocyclique, la diazolidinyl-urée et/ou l'imidazolidinyl-urée, et

- lors de l'étape b), le détergent cationique est sélectionné parmi le groupe défini à la revendication 2 ou une composition de lyse telle que définie à la revendication 3 est utilisée et où la composition comprenant l'échantillon stabilisé ou une fraction de l'échantillon stabilisé, le au moins un détergent cationique et optionnellement un ou plusieurs autres agents de lyse est incubée pour fournir l'échantillon lysé ; et

- l'étape c) comprend les étapes suivantes :

i. mettre en contact l'échantillon lysé ou une portion contenant des acides nucléiques obtenue à partir de l'échantillon lysé avec un ou plusieurs agents de lyse supplémentaires en fournissant ainsi un mélange de lyse, où de préférence à cette fin l'échantillon lysé ou la portion contenant des acides nucléiques obtenue à partir de l'échantillon lysé est mis en contact avec (aa) au moins un agent chaotropique, de préférence un sel chaotropique ; (bb) au moins une enzyme protéolytique ; et/ou (cc) un ou plusieurs sels, en fournissant ainsi un mélange de lyse ;

- optionnellement, extraire l'ADN du mélange de lyse ;

ii. ajouter de l'alcool au mélange de lyse pour ajuster les conditions de liaison et lier l'ARN à une phase solide de liaison d'acide nucléique ;

iii. séparer la phase solide avec l'ARN lié du reste de l'échantillon ; et

iv. optionnellement, laver l'ARN et

v. optionnellement, éluer de l'ARN de la phase solide.

Figure 1a

Figure 1b

Figure 2

Figure 3a

Figure 3b

Figure 4a

Figure 4b

Figure 4c

Figure 5a

Figure 5b

Figure 6

Figure 7a

Figure 7b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6617170 B
- US 7270953 B, Kruhoffer
- US 5460797 A
- US 5459073 A
- US 20110111410 A
- WO 0171732 A
- WO 2004003231 A
- WO 2003004150 A
- WO 9831840 A
- WO 9831461 A

- EP 1260595 A
- WO 9641811 A
- EP 0343934 A
- EP 0880537 A
- WO 9521849 A
- EP 10007346 A
- DE 102005040259
- DE 102005058979
- US 20110111410 A1, Ryan

**Non-patent literature cited in the description**

- **GENOV et al.** *Int. J. Peptide Protein Res.,* 1995, vol. 45, 391-400
- handbook. September 2010
- handbook. April 2010

- instructions of the handbook. April 2008
- kit handbook. April 2008
- handbook. November 2005